# EUROPEAN PATENT SPECIFICATION

(11) **EP 3 556 851 B1**
(45) Date of publication and mention of the grant of the patent: **12.11.2025**
(21) Application number: 17846664.5
(22) Date of filing: 31.08.2017
(51) Int. Cl.: C12N 5/0784, C12N 5/0783, C12N 5/0786, A61K 40/10, A61K 40/19, A61K 40/24, A61K 40/42, A61K 40/15

(54) **METHOD FOR MANUFACTURING NATURAL-KILLER-T (NKT)-CELL STIMULATING DENDRITIC CELL AND METHOD FOR MANUFACTURING CELL COMPOSITION CONTAINING NKT-CELL STIMULATING DENDRITIC CELL AND NKT CELL**
VERFAHREN ZUR HERSTELLUNG VON DENDRITISCHEN ZELLEN ZUR STIMULIERUNG EINER NATURAL-KILLER-T (NKT)-ZELLE UND VERFAHREN ZUR HERSTELLUNG DER ZUSAMMENSETZUNG MIT DENDRITISCHEN ZELLEN ZUR STIMULIERUNG EINER NKT-ZELLE SOWIE NKT-ZELLE
PROCÉDÉ DE PRODUCTION D'UNE CELLULE DENDRITIQUE STIMULANT LES LYMPHOCYTES T TUEURS NATURELS (NKT) ET PROCÉDÉ DE PRÉPARATION D'UNE COMPOSITION CELLULAIRE CONTENANT UNE CELLULE DENDRITIQUE STIMULANT LES LYMPHOCYTES NKT ET LYMPHOCYTE NKT

(30) Priority: 01.09.2016 JP 2016170996
(43) Date of publication of application: 23.10.2019
(73) Proprietor: Riken Immune Regenerative Medicine Inc., Tokyo 102-0082 (JP)
(72) Inventor: NOGUCHI, Katsuo, Tokyo 150-0013 (JP); TADAKI, Toshio, Tokyo 150-0013 (JP)
(74) Representative: Kuhnen & Wacker Patent- und Rechtsanwaltsbüro PartG mbB
(86) International application number: PCT/JP2017/031419
(87) International publication number: WO 2018/043660

(56) References cited:
- WO-A1-2009/034961
- WO-A1-2015/129791
- JP-A- 2009 518 045
- US-A1- 2016 361 358
- CHANG D H ET AL: "Sustained expansion of NKT cells and antigen-specific T cells after injection of alpha-galactosyl-ceramide loaded mature dendritic cells in cancer patients", THE JOURNAL OF EXPERIMENTAL MEDICINE, ROCKEFELLER UNIVERSITY PRESS, US, vol. 201, no. 9, 1 May 2005 (2005-05-01), pages 1503 - 1517, XP003026818, ISSN: 0022-1007
- S. MOTOHASHI ET AL: "A Phase I-II Study of ?-Galactosylceramide-Pulsed IL-2/GM-CSF-Cultured Peripheral Blood Mononuclear Cells in Patients with Advanced and Recurrent Non-Small Cell Lung Cancer", THE JOURNAL OF IMMUNOLOGY, vol. 182, no. 4, 15 February 2009 (2009-02-15), pages 2492 - 2501, XP055386155, ISSN: 0022-1767, DOI: 10.4049/jimmunol.0800126
- PROMOCELL GMBH: "Generation of monocyte-derived dendritic cells (moDCs)", APPLICATION NOTE, February 2015 (2015-02-01), pages 1 - 7, XP055586343, Retrieved from the Internet <URL:https://www.promocell.com/f/2017/11/Generation_of_monocyte-derived_Dendritic_Cells-1.pdf>

## Description

### TECHNICAL FIELD

The present invention relates to a method for manufacturing NKT cell-stimulating dendritic cells, and a method for manufacturing a cell composition containing NKT cell-stimulating dendritic cells and NKT cells and NKT cells.

### BACKGROUND ART

Among recent methods for treating cancer, in addition to surgery, chemotherapy, and radiation therapy, immunotherapy involving extracting immune cells from a patient, activating the cells, and subsequently returning the cells to the patient has been gaining attention. A reduction in the number of immune cells in the body or a decrease in immune cell activity may be a cause leading to the onset of cancer. In immunotherapy, this is overcome by increasing the number of immune cells or the like so as to attack cancer cells.

In one known example of a method for cancer immunotherapy, cancer is treated by pulsing peripheral mononuclear cells with α-galactosylceramide to activate or proliferate NKT cells which are included in the mononuclear cells, and then administering the NKT cells to the patient. Clinical research of this therapy with a focus on lung cancer has been progressing (for example, refer to Literature 1).

NKT cells are a subgroup of T cells that possesses the characteristics of both T cells and natural killer cells (NK cells). NKT cells make up only about 0.1% of T cells in peripheral blood. A defining feature of NKT cells is that when activated, they produce large amounts of IFN-y, IL-4, and granulocyte macrophage colony-stimulating factor (GM-CSF).

It is known that NKT cells are activated upon transmission of an activation signal via a T cell receptor Vα24Vβ11 on the surface of the NKT cells when α-galactosylceramide (α-GalCer) is presented by a CD-1d molecule on the surface of an antigen-presenting cell (dendritic cell).

Moreover, Literature 2 discloses a pharmaceutical containing dendritic cells, and a method for producing the same according to prior art.

In addition, Literature 3 teaches about sustained expansion of NKT cells and antigen-specific T cells after injection of α-galactosyl-ceramide loaded mature dendritic cells in cancer patients.

### CITATION LIST

### LITERATURE

Literature 1: Shinichiro Motohashi, Kaoru Nagato, Naoki Kunii, Heizaburo Yamamoto, Kazuki Yamasaki, Kohsuke Okita, Hideki Hanaoka, Naomi Shimizu, Makoto Suzuki, Ichiro Yoshino, Masaru Taniguchi, Takehiko Fujisawa, and Toshinori Nakayama, A Phase I-II Study of α-Galactosylceramide-Pulsed IL-2/GM-CSF-Cultured Peripheral Blood Mononuclear Cells in Patients with Advanced and Recurrent Non-Small Cell Lung Cancer1, The Journal of Iummnolog 182:2492-2501 (2009)
Literature 2: WO 2015/129791 A1
Literature 3: Chang et al., The Journal of Experimental Medicine, Vol. 201, No. 9, 2005, pages 1503 to 1517

### SUMMARY OF INVENTION

### PROBLEM SOLVED BY THE INVENTION

No methods for efficiently and economically manufacturing NKT cell-stimulating dendritic cells have been known in the past.

Thus, a problem of the present invention is to provide a method for economically manufacturing NKT cell-stimulating dendritic cells.

### MEANS FOR SOLVING THE PROBLEM

One embodiment of the present invention is a method for manufacturing NKT cell-stimulating dendritic cells. This manufacturing method is characterized by including the following steps:
(a1) an adhering step in which mononuclear cells are placed into a culture vessel and the culture vessel is kept still so as to allow some of the mononuclear cells to adhere to a bottom surface of the vessel;
(b1) a removal step in which suspended cells other than the cells which have adhered to the bottom surface of the culture vessel are removed;
(c1) a differentiation step in which both of IL-4 and granulocyte macrophage colony-stimulating factor are added into the culture vessel so as to cause monocytes among the cells which have adhered to the bottom surface to differentiate into immature dendritic cells;
(d1) a maturation step in which IL-4, granulocyte macrophage colony-stimulating factor, tumor necrosis factor α, and prostaglandin E2 are added into the culture vessel so as to mature the immature dendritic cells; and
(e1) an induction step in which α-galactosylceramide is added into the culture vessel so as to induce NKT cell-stimulating dendritic cells from the mature dendritic cells.

According to this embodiment, NKT cell-stimulating dendritic cells can be obtained efficiently, in a relatively short amount of time, and with a small amount of culture medium by causing monocytes among the mononuclear cells to adhere to the bottom surface of the culture vessel and differentiating these monocytes into dendritic cells. Therefore, the manufacturing method of the present embodiment enables NKT-stimulating dendritic cells to be prepared more economically and with higher precision than, for example, the method for obtaining NKT-stimulating dendritic cells disclosed in Literature 1.

Another embodiment of the present invention is a method for manufacturing a cell composition containing NKT cell-stimulating dendritic cells and NKT cells. This manufacturing method is characterized by including the following steps:
(a2) an adhering step in which mononuclear cells are placed into a first culture vessel and the first culture vessel is kept still so as to allow some of the mononuclear cells to adhere to a bottom surface of the vessel;
(b2) a collection step in which suspended cells other than the cells which have adhered to the bottom surface of the vessel are collected and preserved;
(c2) a differentiation step in which both of IL-4 and granulocyte macrophage colony-stimulating factor are added into the first culture vessel so as to cause monocytes among the cells which have adhered to the bottom surface to differentiate into immature dendritic cells;
(d2) a maturation step in which IL-4, granulocyte macrophage colony-stimulating factor, tumor necrosis factor α, and prostaglandin E2 are added into the first culture vessel so as to mature the immature dendritic cells;
(e2) a dendritic cell induction step in which α-galactosylceramide is added into the first culture vessel so as to induce NKT cell-stimulating dendritic cells from the mature dendritic cells; and
(f2) an NKT cell induction step in which the NKT cell-stimulating dendritic cells and the preserved suspended cells are placed in a second culture vessel and the cells are cultured so as to induce NKT cells from some of the suspended cells..

In this embodiment, the suspended cells which did not adhere to the bottom surface of the culture vessel are collected, and then mixed and cultured with the NKT cell-stimulating dendritic cells in a subsequent step. Thereby, NKT cells can be induced from some of the suspended cells, and a cell composition containing NKT cell-stimulating dendritic cells and NKT cells can be obtained.

The method for manufacturing NKT cell-stimulating dendritic cells according to the above embodiment may further include a step for cryopreserving the obtained NKT cell-stimulating dendritic cells.

Further, the method for manufacturing a cell composition according to the above embodiment may also further include a step for cryopreserving the obtained cell composition.

Further, the galactosylceramide concentration for inducing the cell in the method for manufacturing the cell composition may be in the range from 10 to 1,000 ng/mL.

### ADVANTAGEOUS EFFECTS OF INVENTION

According to the present invention, NKT cell-stimulating dendritic cells can be provided economically and with high precision in a relatively short amount of time.

### BRIEF DESCRIPTION OF DRAWINGS

FIG. 1 shows the results on Day 0, Day 7, and Day 14 upon analyzing NKT cells using a flow cytometer in a culture experiment of Example 1.
FIG. 2 shows the results on Day 0, Day 7, and Day 14 upon analyzing NKT cells using a flow cytometer in a culture experiment of Example 1.
FIG. 3 shows the results on Day 0, Day 7, and Day 14 upon analyzing NKT cells using a flow cytometer in a culture experiment of Example 1.
FIG. 4 shows the total number of cells and the viable cell ratio in the experiment group of Example 1.
FIG. 5 shows the rate of differentiation of dendritic cells in the experiment group of Example 1.
FIG. 6 shows the results on Day 0, before freezing, and after freezing and thawing upon analyzing CD14, CD83, and CD86 on the cell surface using a flow cytometer in a culture experiment of Example 2.
FIG. 7 shows the results on Day 0, before freezing, and after freezing and thawing upon analyzing CD14, CD83, and CD86 on the cell surface using a flow cytometer in a culture experiment of Example 2.
FIG. 8 shows the results on Day 0, before freezing, and after freezing and thawing upon analyzing CD14, CD83, and CD86 on the cell surface using a flow cytometer in a culture experiment of Example 2.
FIG. 9 shows the total number of cells and the viable cell ratio before freezing and after freezing and thawing in the experiment group of Example 2.
FIGS. 10(A) to 10(C) show the results on Day 0, Day 2, and Day 6 upon analyzing NKT cells using a flow cytometer in a culture experiment of Example 3.
FIGS. 11(A) to 11(C) show the results on Day 0, Day 2, and Day 6 upon analyzing NKT cells using a flow cytometer in a culture experiment of Example 3.
FIGS. 12(A) to 12(C) show the results on Day 0, Day 2, and Day 6 upon analyzing NKT cells using a flow cytometer in a culture experiment of Example 3.
FIGS. 13(A) and 13(B) show the results on Day 0 and Day 6 upon analyzing dendritic cells using a flow cytometer in a culture experiment of Example 3.
FIGS. 14(A) and 14(B) show the results on Day 0 and Day 6 upon analyzing dendritic cells using a flow cytometer in a culture experiment of Example 3.
FIGS. 15(A) and 15(B) show the results on Day 0 and Day 6 upon analyzing dendritic cells using a flow cytometer in a culture experiment of Example 3.
FIG. 16 shows the total number of cells and the viable cell ratio in the experiment group of Example 3.

### DESCRIPTION OF EMBODIMENTS

Embodiments of the present invention will now be explained below. In these embodiments, the prior art and conventionally well-known technology may be invoked and appropriately designed/modified.

In an embodiment of the manufacturing method of the present invention, first, mononuclear cells are placed into a culture vessel and the culture vessel is kept still so as to allow some of the mononuclear cells to adhere to a bottom surface of the vessel. Herein, many monocytes are included among the cells which have adhered to the bottom surface of the vessel. By isolating the monocytes in this way, the monocytes can be efficiently differentiated to dendritic cells, and ultimately NKT cell-stimulating dendritic cells can be efficiently obtained in a relatively short amount of time. Further, in this embodiment, since the monocytes are isolated as described above, the amount of culture medium to be used can be reduced, and thus the NKT cell-stimulating dendritic cells can be manufactured at a lower cost.

In this specification, the term "mononuclear cells" refers to a cell population which includes lymphocytes and monocytes. Mononuclear cells can be obtained from blood, such as peripheral blood, bone marrow, and cord blood, using a publicly-known method such as centrifugation and flow cytometry. Further, mononuclear cells can be directly collected from a human being by apheresis. It is preferable to obtain the mononuclear cells by apheresis because the process can be simplified and there are few effects on the subjects given that the blood components other than the mononuclear cells can be returned.

Culture medium is added to the culture vessel. For the culture medium, a publicly-known culture medium suited to culturing mononuclear cells can be used. For example, a culture medium obtained by appropriately adding additives such as serum, growth factors, cytokines, serum substitute, vitamins, buffers, and inorganic salts to a basic culture medium used in culturing animal cells (for example, CELL GRO GMP Serum-free Dendritic Cell Medium (Cell Genix, #20801-0500), etc.) can be used. As concrete examples of the culture medium, mention may be made of, but not limited to, ALyS505N-0 (NIPRO, #1020P10), AIM-V@Medium CTS (ThermoFisher, #0870112-DK), etc.

The culture vessel to be used is not particularly limited, as long as the vessel is generally used for culturing animal cells and has a bottom surface to which cells can be adhered. As non-limiting examples of the culture vessel, mention may be made of a commercially-available plate (AGC TECHNO GLASS, #3810-006), dish (CRNING, CellBIND Surface 100 mm, #3296), flask (Sumitomo Bakelite, #MS-21050), etc.

The duration of time for keeping the culture vessel into which the mononuclear cells have been placed still so as to allow some of the mononuclear cells to adhere to the bottom surface of the vessel is not particularly limited, but may be, for example, 1 to 12 hours, preferably 2 hours. The temperature and atmosphere while keeping the culture vessel still may be conditions which are used for culturing mononuclear cells, and such conditions can be set using an incubator, etc. which is normally used for culturing. For example, the temperature may be about 35 to 38°C, preferably 37°C, and the CO₂ concentration may be about 1 to 10%, preferably about 5%.

Removal of suspended cells which have not adhered to the bottom surface of the vessel may be performed by a normal method. For example, the suspended cells can be removed by suction. The suspended cells that are removed may be discarded or preserved. Namely, in another embodiment of the present invention, the suspended cells which have not adhered to the bottom surface of the vessel may be collected and preserved. For example, the suspended cells which have been collected can be suspended in a cryopreservation liquid suited to cryopreserving cells, placed in a cryopreservation vessel, and then cryopreserved at -80°C. As an example of the cell cryopreservation liquid, mention may be made of CP-1 (Kyokuto Pharmaceutical Industrial Co., Ltd., #324042-3), etc.

Next, culture medium and a prescribed factor(s) are placed into the culture vessel in which cells have adhered to the bottom surface thereof so as to cause monocytes among the cells which have adhered to the bottom surface to differentiate into immature dendritic cells. The culture mediums explained above can be used as the culture medium. As the predetermined factor(s) for causing the monocytes to differentiate into immature dendritic cells, mention may be made of IL-4 and GM-CSF, and a combination of both of these factors is preferable. If IL-4 is used, the concentration range thereof is preferably 100 to 1,000 ng/mL, more preferably 200 to 800 ng/mL, and even more preferably 400 to 600 ng/mL. If GM-CSF is used, the concentration range thereof is preferably 100 to 1,000 ng/mL, more preferably 200 to 800 ng/mL, and even more preferably 400 to 600 ng/mL.

In the culturing for differentiation, the same conditions as the culture conditions described above can be used, and these conditions may be appropriately modified by a person skilled in the art. Further, the culturing time should be sufficient for causing the monocytes to differentiate into immature dendritic cells, and may be appropriately set by a person skilled in the art. For example, the culturing time is 4 to 6 days, preferably 5 days.

Next, a prescribed factor(s) is placed into the culture vessel so as to mature the immature dendritic cells. As the prescribed factor(s) to be used herein, mention may be made of IL-4, GM-CSF, Tumor Necrosis Factor α (TNFα), and Prostaglandin 2 (PGE₂). A combination of these factors is preferable. If IL-4 is used, the concentration range thereof is preferably 100 to 1,000 ng/mL, more preferably 200 to 800 ng/mL, and even more preferably 400 to 600 ng/mL. If GM-CSF is used, the concentration range thereof is preferably 100 to 1,000 ng/mL, more preferably 200 to 800 ng/mL, and even more preferably 400 to 600 ng/mL. If TNFα is used, the concentration range thereof is preferably 1.0 to 100 ng/mL, more preferably 5.0 to 20 ng/mL, and even more preferably 8 to 12 ng/mL. If PGE₂ is used, the concentration range thereof is preferably 100 to 10,000 ng/mL, more preferably 500 to 5,000 ng/mL, and even more preferably 800 to 1,200 ng/mL.

The culture conditions for maturing may be the same conditions as the culture conditions described above, and these conditions may be appropriately modified by a person skilled in the art. The culturing time should be sufficient for allowing the immature dendritic cells to mature, and may be appropriately set by a person skilled in the art. For example, the culturing time is 1 to 3 days, preferably 2 days.

Next, α-galactosylceramide (α-GalCer) is added to the culture vessel to induce NKT cell-stimulating dendritic cells from the matured dendritic cells. Herein, "NKT cell-stimulating dendritic cells" indicate dendritic cells that induce activation and proliferation of NKT cells. On the surface of these dendritic cells, α-GalCer is presented by CD-1d molecules, and NKT cells are activated when the NKT cells recognize the α-GalCer.

The concentration range of α-GalCer in this step is preferably 10 to 1,000 ng/mL, more preferably 20 to 800 ng/mL, and even more preferably 80 to 120 ng/mL.

In this step, the same conditions as the culture conditions described above can be used for the culture conditions for obtaining the NKT cell-stimulating dendritic cells, and these conditions may be appropriately modified by a person skilled in the art. The culturing time should be sufficient for causing the matured dendritic cells to become NKT cell-stimulating dendritic cells, and may be appropriately set by a person skilled in the art. For example, the culturing time is 0.25 to 2 days, preferably 1 day.

In another embodiment, in the case that the suspended cells which did not adhere to the bottom surface are collected and preserved, after the NKT cell-stimulating dendritic cells are obtained as described above, these dendritic cells and the suspended cells are placed into a different culture vessel and cultured, and thereby NKT cells can be induced from some of the suspended cells. Herein, if the suspended cells that were collected were cryopreserved, the suspended cells can be thawed at, for example, 37°C and then mixed with the dendritic cells. In the present embodiment, the NKT cell-stimulating dendritic cells cause some of the suspended cells to differentiate to NKT cells and become activated. Thereby, in the present embodiment, a cell composition containing NKT cell-stimulating dendritic cells and NKT cells can be obtained. Herein, in this specification, the term "cell composition" indicates a composition that contains cells and that also contains components other than cells such as culture medium, preservation liquid, etc.

The manufacturing methods of the above-described embodiments may further include a step for cryopreserving the NKT cell-stimulating dendritic cells that were obtained or the cell composition containing these dendritic cells and NKT cells. The cell survival rate and the expression of differentiation markers do not decrease even if the dendritic cells or the cell composition is cryopreserved, and thus there is no loss of cell function. Since cryopreservation is possible as described above, the NKT cell-stimulating dendritic cells and the cell composition containing NKT cell-stimulating cells and NKT cells can be stably provided. The cell cryopreservation can be carried out by cell cryopreservation methods known to those skilled in the art. For example, the cell cryopreservation can be carried out by suspending the cells in a publicly-known preservation liquid that is suited to freezing cells, freezing the suspension solution with, for example, liquid nitrogen, and preserving the frozen suspension solution at, for example, -80°C or less.

Also disclosed herein are the NKT cell-stimulating dendritic cells and the cell composition containing the dendritic cells and NKT cells obtained by the manufacturing methods of the above-described embodiments possess an immune-stimulating action and an anticancer action. A cell preparation for use in infectious disease treatment or cancer immunotherapy as disclosed herein can be manufactured by mixing the dendritic cells or the cell composition with a pharmaceutically-acceptable carrier.

In the following, the present invention will be explained in further detail by way of examples, but the scope of the present invention is not limited to the examples explained below.

### EXAMPLES

The present inventors manufactured NKT cell-stimulating dendritic cells and a cell composition containing the dendritic cells and NKT cells as described below using blood samples (ID: NKT001-009) from healthy individuals.

### (Materials)

The materials used in the following experiments are as follows.
- α-GalCer: Osaka Synthetic Chemical Laboratories, Inc. (Osaka Synthetic), Lot No. N-32-A (GMP-grade product)
- GM-CSF: Miltenyi Biotec, #170-076-136
- IL-4: Miltenyi Biotec, #170-076-135
- IL-2: NIPRO, #87890
- Prostaglandin E-2: SIGMA, #P6532-1MG
- TNF-alpha: Miltenyi Biotec, #170-076-103
- culture medium: ALyS505N-0 (NIPRO, #1020P10)
   AIM-V@Medium CTS (ThermoFisher, #0870112-DK)
- antibodies:
   FITC Mouse IgG1, κ Isotype Control (FC) Catalog #400114 BioLegend FITC Mouse IgG2a, κ Isotype Control (FC) Catalog #400214 BioLegend PE/Cy7 Mouse IgG1, κ Isotype Control (FC) Catalog #400126 BioLegend APC/Cy7 Mouse IgG1, κ Isotype Control (FC) Catalog #400128 BioLegend Anti-TCR Vα24-FITC Catalog # IM1589 BECKMAN COULTER Anti-TCR Vβ11-PE Catalog # IM2290 BECKMAN COULTER PE/Cy7 anti-human CD3 Catalog # 300420 BioLegend APC/Cy7 anti-human CD56 (NCAM) Catalog # 318332 BioLegend

### (Example 1)

On the first day (Day 0), the cell components were first isolated by apheresis. The cell components were layered in layers of 30 mL each in a LeucoSep, and centrifugation (3000 rpm, 20 min, r/t, Accel: 1, Decel: 1) was carried out. Thereafter, the mononuclear cell layer was collected in a 50 mL centrifuge tube, an equivalent amount of physiological salt solution was added thereto to create a suspension, and then centrifugation (1500 rpm, 5 min, r/t, Accel: 3, Decel: 2) was carried out. 30 mL of AIM-V was added to create a suspension, and then the cell aggregate was removed using a cell strainer and the number of cells was measured. 2 × 10⁷ mononuclear cells were sampled (for Day 0) for use in flow cytometer measurement. The mononuclear cells were seeded into six 225 cm² suspension culture flasks to which 30 mL AIM-V was added, the flasks were kept still for two hours in an incubator (37°C, 5% CO₂), and non-adhered cells were collected. The non-adhered cells that were collected were centrifuged (1500 rpm, 5 min, r/t, Accel: 3, Decel: 3), and AIM-V was added to reach 2 × 10⁸ cells in a suspension. An equivalent amount of CP-1 containing 11.8% human serum albumin was added, and then the non-adhered cell suspension was placed in a flowbag (NIPRO) and preserved at -80°C. ALyS505N-0 (NIPRO, #1020P10) was used as the culture medium.

Meanwhile, 30 mL of AIM-V was added to the adhered cells in the flasks, and to the AIM-V in the flasks, IL-4 was added to reach 500 U/mL and GM-CSF was added to reach 500 U/mL, and then the flasks were kept still for 5 days in an incubator (37°C, 5% CO₂).

On the fifth day from the start of culturing, to the AIM-V in the flasks, IL-4 was added to reach 500 U/mL, GM-CSF was added to reach 500 U/mL, TNFα was added to reach 10 ng/mL, and PGE₂ was added to reach 1 µg/mL, and then the flasks were kept still for 1 day in an incubator (37°C, 5% CO₂). On the sixth day from the start of culturing, to the AIM-V in the flasks, α-GalCer was added to reach 100 ng/mL, and the flasks were kept still for a further 1 day in an incubator (37°C, 5% CO₂).

On the seventh day from the start of culturing, suspended cells were collected from the flasks, centrifuged (1500 rpm, 5 min, r/t, Accel: 3, Decel: 3), and suspended by adding 30 mL of physiological salt solution. Centrifugation (1500 rpm, 5 min, r/t, Accel: 3, Decel: 2) was carried out again, and 30 mL of ALyS505N-0 (containing 100 U/mL of IL-2) was added to create a suspension. Herein, the non-adhered cells preserved on Day 0 were thawed at 37°C, centrifuged (1500 rpm, 5 min, r/t, Accel: 3, Decel: 2), and 30 mL of ALyS505N-0 (containing 100 U/mL of IL-2) was added to create a suspension. These cell groups were seeded into bags (with 2L of culture medium) obtained by connecting two 1L bags with ALyS505N-0 (containing 100 U/mL of IL-2) using a connecting tube, and the bags were kept still for 1 day in an incubator (37°C, 5% CO₂). 1 mL was sampled from the seeded bags for ELISA measurement and then centrifuged (1500 rpm, 5 min, r/t, Accel: 3, Decel: 3). Therein, the cell concentration and viable cell ratio of the cells cultured in the 2L bags were measured. Further, the cell surface molecules were measured with a flow cytometer using some of the cells that were cultured in the 2L bags.

NKT cell confirmation was conducted with TCR Vα24-FITC or TCR Vα24-PerCP/Cy5.5, TCR Vβ11-PE, CD3-PE/Cy7, CD56-APC or CD56-APC/Cy7, and dendritic cell confirmation was conducted with CD14-PE, CD83-PE, CD80-PE or CD86-PE. The remaining cells in the 2L bags were further continuously cultured for 1 to 2 weeks. Flow cytometer analysis was conducted to confirm the NKT cells in the continuously cultured cells.

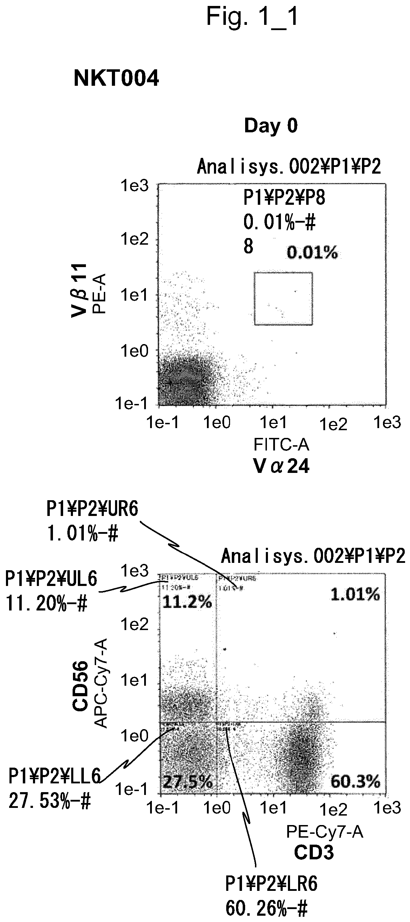

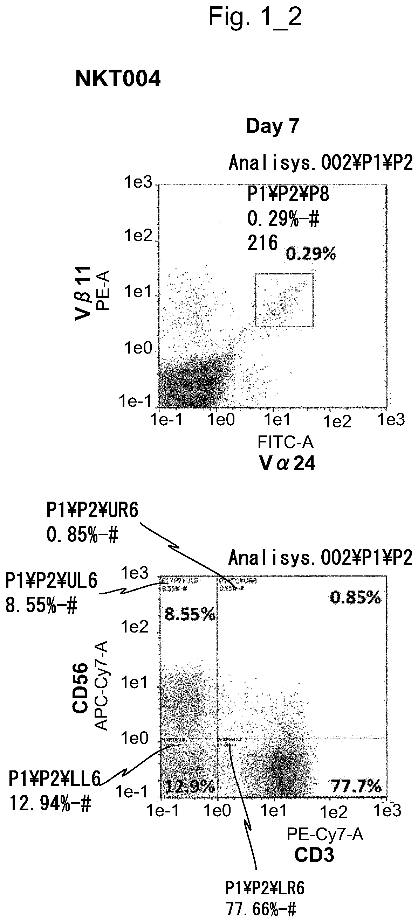

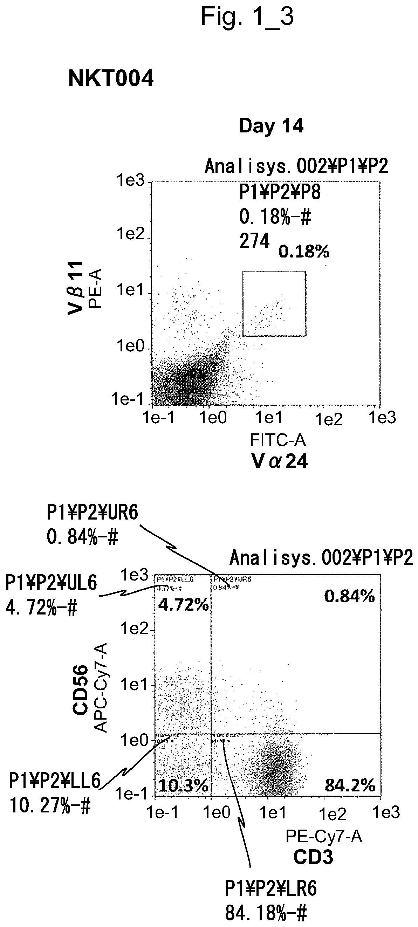

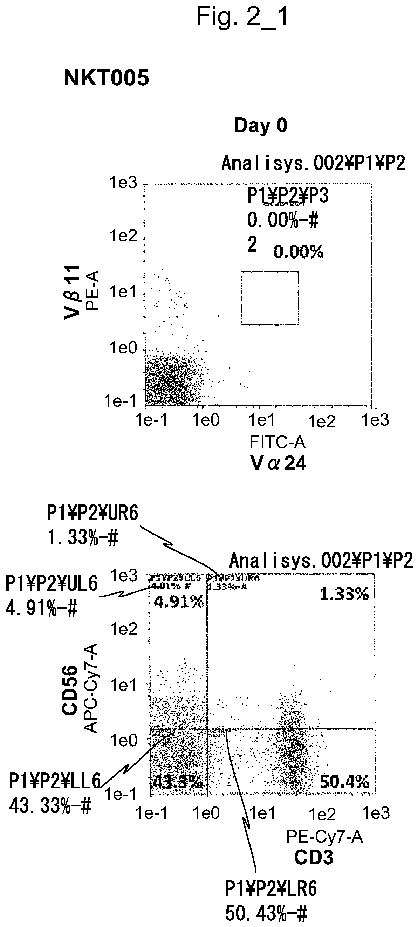

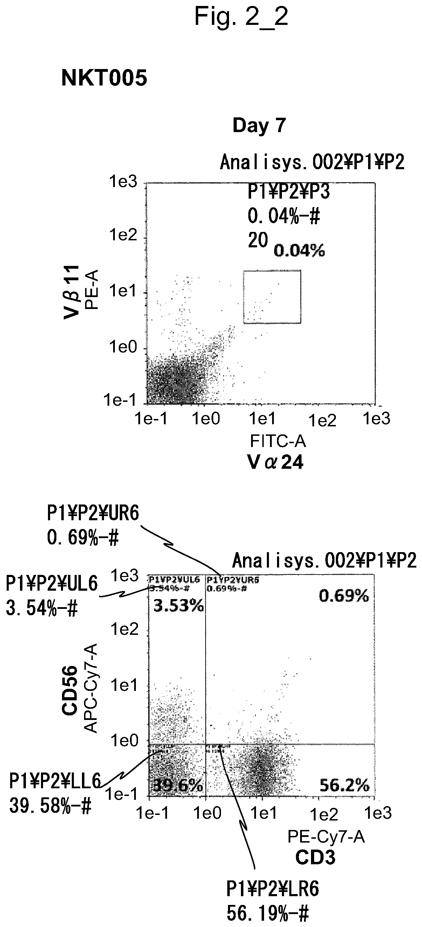

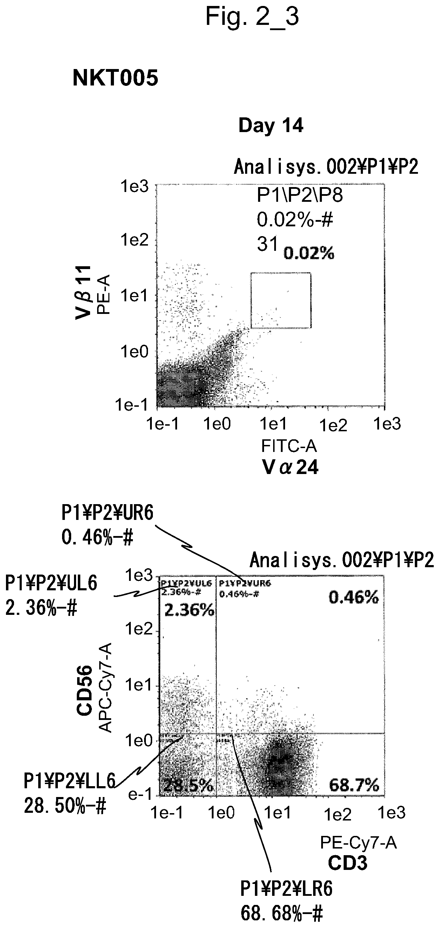

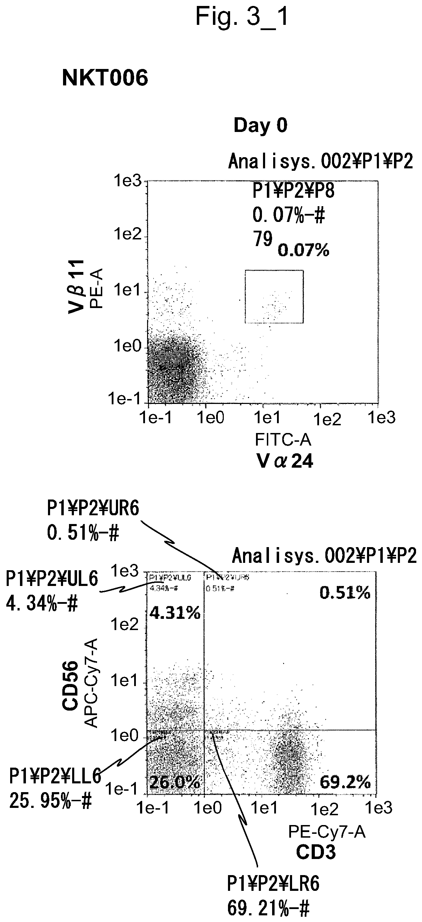

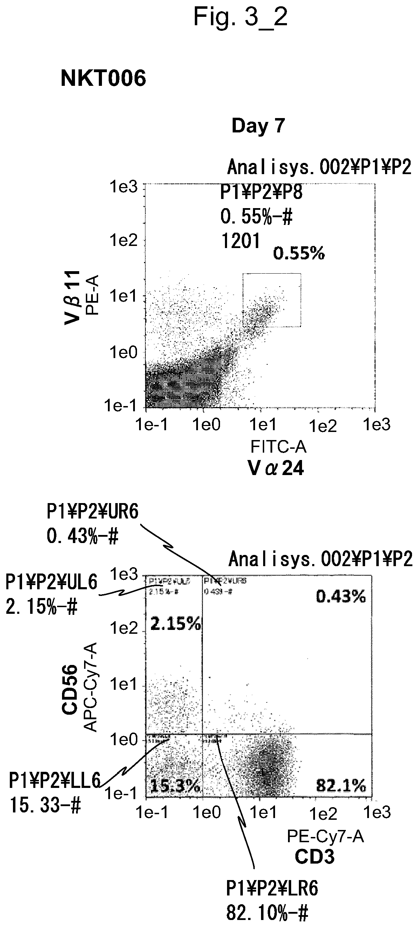

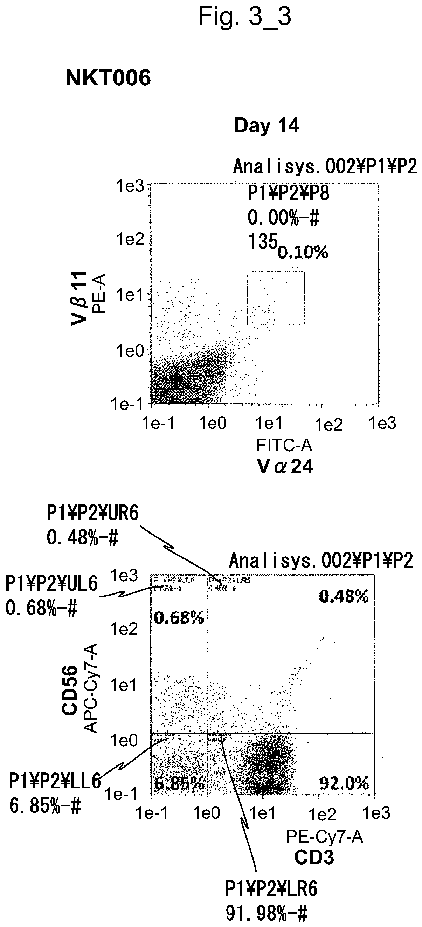

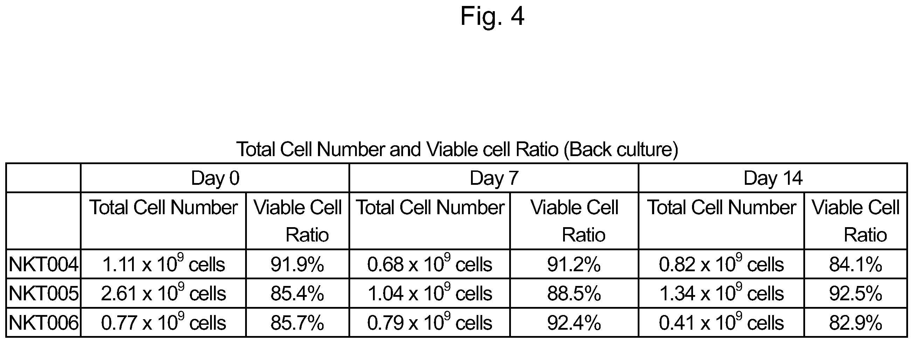

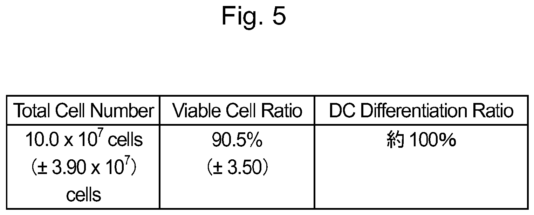

The flow cytometer analysis results are shown in FIGS. 1 to 3. FIGS. 1 to 3 illustrate the results upon conducting the same experiment using cells obtained from three healthy individuals (ID: NKT004, NKT005, and NKT006). Va24 and Vb11 in these drawings are the two subunits of a T-cell receptor (TCR) which is an essential molecule for antigen recognition, and are also the TCR subunits that recognize α-GalCer. If fluorescent antibodies that specifically bind to these subunits are mixed with a cell group to be inspected and then flow cytometer analysis is carried out, the cells to which the antibody that recognizes Va24 has adhered shift in the horizontal axis direction and the cells to which the antibody that recognizes Vb11 has adhered shift in the vertical axis direction, and the fluorescence intensity is strongly detected. Cells to which both of these antibodies have bonded appear as dots in the square in the center of the graph. Each of these dots represents an NKT cell. The numerical value (%) that is indicated in the drawings is the ratio of NKT cells occupying the total number of cells used in a single analysis. In all of the experiments, the ratio of NKT cells reached a maximum at one week from the start of culturing. FIG. 4 shows the cell survival rate in this group of experiments. FIG. 5 shows the total number of dendritic cells after culture, the viable cell ratio, and the rate of differentiation to dendritic cells (DC) in this group of experiments.

As is clear from FIGS. 1 to 3 and 5, prominent NKT cell proliferation (reaching at maximum about 30-fold) was confirmed after 1 week of culturing upon isolating monocytes by making the monocytes among the mononuclear cells adhere to the bottom surface of the flask and then causing the monocytes to differentiate into dendritic cells. It was also confirmed that the NKT cell ratio tended to decrease after two weeks. During the culture period, there was no change in the total number of cells, and there was no decrease in the viable cell ratio (FIG. 4).

### (Example 2)

On the first day (Day 0), the cell components were first isolated by apheresis. The cell components were layered in layers of 30 mL each in a LeucoSep, and centrifugation (3000 rpm, 20 min, r/t, Accel: 1, Decel: 1) was carried out. The mononuclear cell layer was collected in a 50 mL centrifuge tube, an equivalent amount of physiological salt solution was added thereto to create a suspension, and then centrifugation (1500 rpm, 5 min, r/t, Accel: 3, Decel: 2) was carried out. 30 mL of AIM-V was then added to create a suspension, and the cell aggregate was removed using a cell strainer and the number of cells was measured. 2 × 10⁷ mononuclear cells were sampled (for Day 0) for use in flow cytometer measurement. These cells were measured for CD14, CD83, and CD86 with a flow cytometer. The mononuclear cells were seeded into six 225 cm² suspension culture flasks to which 30 mL AIM-V was added, and the flasks were kept still for two hours in an incubator (37°C, 5% CO₂). The non-adhered cells were subsequently discarded, and 30 mL AIM-V was added to the adhered cells in the flasks. To the AIM-V in the flasks, IL-4 was added to reach 500 U/mL and GM-CSF was added to reach 500 U/mL, and then the flasks were kept still for 5 days in an incubator (37°C, 5% CO₂).

On the fifth day after the start of culturing, to the AIM-V in the flasks, IL-4 was added to reach 500 U/mL, GM-CSF was added to reach 500 U/mL, TNF-α was added to reach 10 ng/mL, and PGE₂ was added to reach 1 µg/mL, and then the flasks were kept still for 1 day in an incubator (37°C, 5% CO₂). On the sixth day from the start of culturing, to the AIM-V in the flasks, α-GalCer was added to reach 100 ng/mL, and the flasks were kept still for 1 day in an incubator (37°C, 5% CO₂).

On the seventh day from the start of culturing, suspended cells were collected from the flasks, centrifuged (1500 rpm, 5 min, r/t, Accel: 3, Decel: 3), and suspended by adding 30 mL of physiological salt solution, and then centrifugation (1500 rpm, 5 min, r/t, Accel: 3, Decel: 2) was carried out again. The precipitate cells were suspended by adding 30 mL physiological salt solution and then centrifuged (1500 rpm, 5 min, r/t, Accel: 3, Decel: 2), and the same operation of suspending by adding 30 mL physiological salt solution was repeated two more times. A portion of the cell suspension liquid that was obtained was sampled, and the cell concentration and viable cell ratio thereof were measured. Further, the CD14, CD83, and CD86 on the cell surface were measured with a flow cytometer. Subsequently, centrifugation (1500 rpm, 5 min, r/t, Accel: 3, Decel: 2) was carried out and the collected cells were suspended in 6 mL cryopreservation liquid (10% DMSO, 90% autoserum), and this was dispensed into vials in an amount of 1 mL each. The vials were stored at -80°C. After thawing, the CD14, CD83, and CD86 on the cell surface were measured with a flow cytometer.

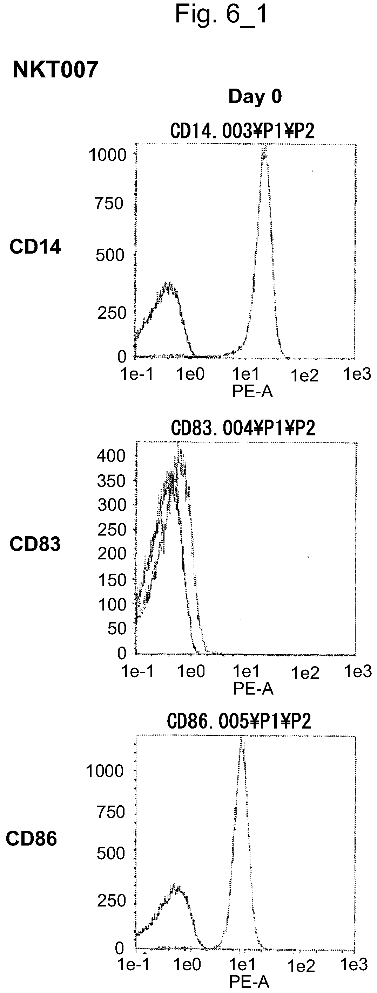

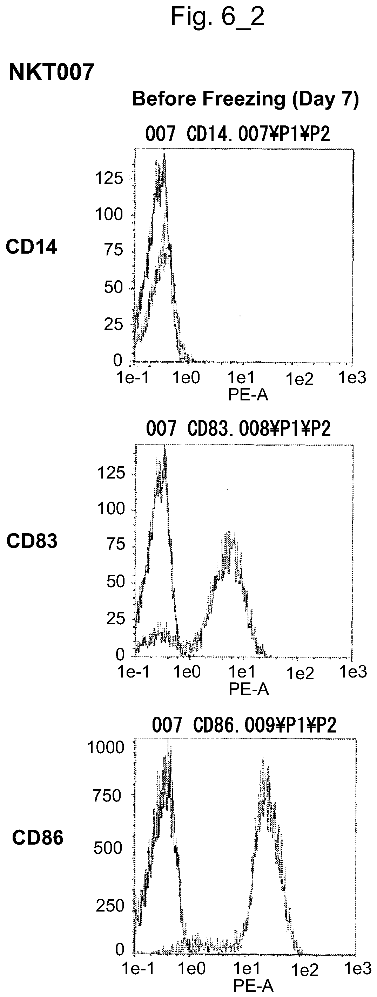

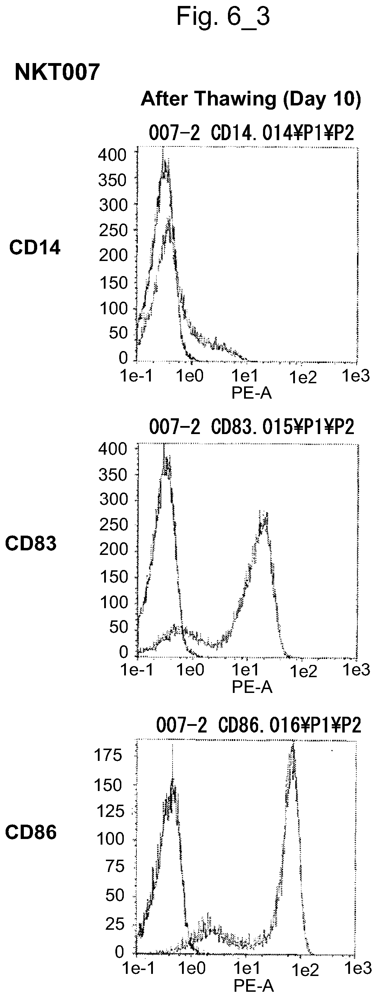

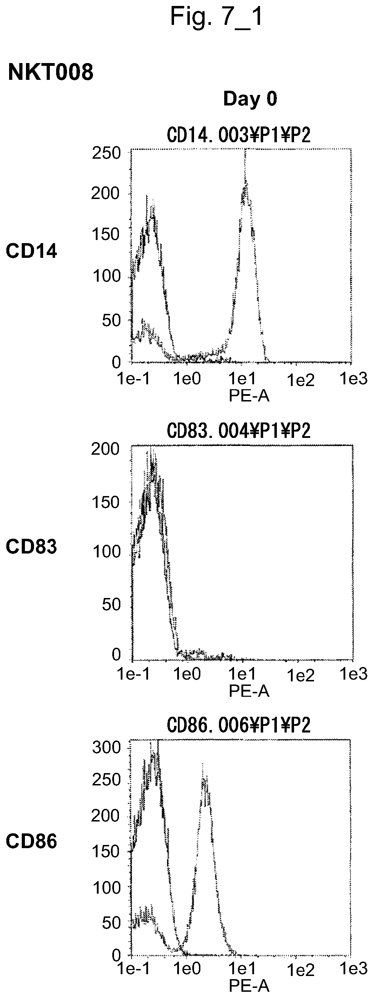

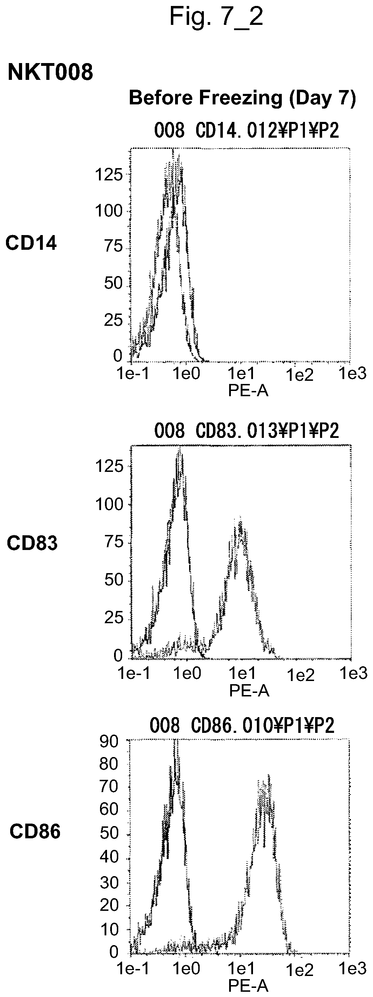

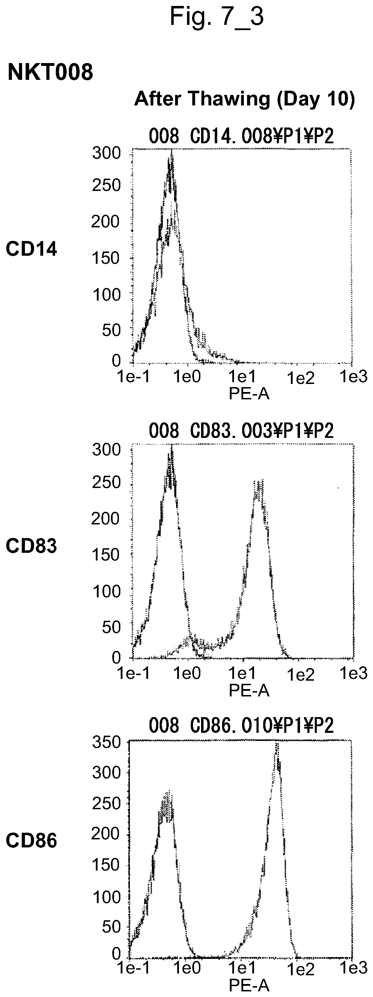

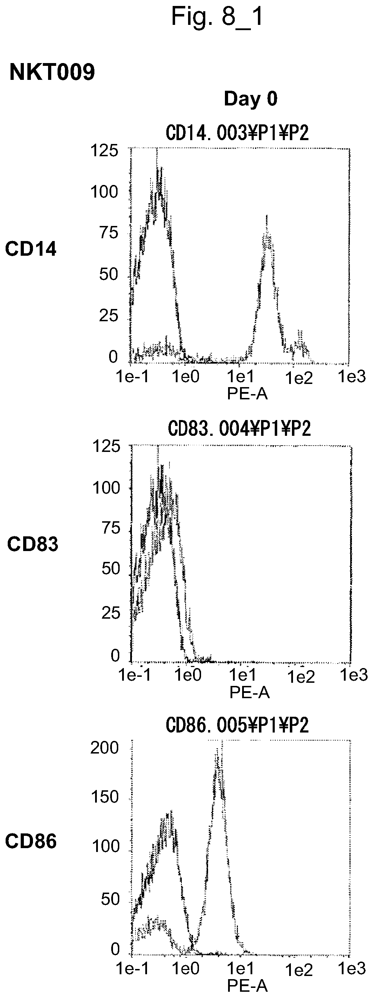

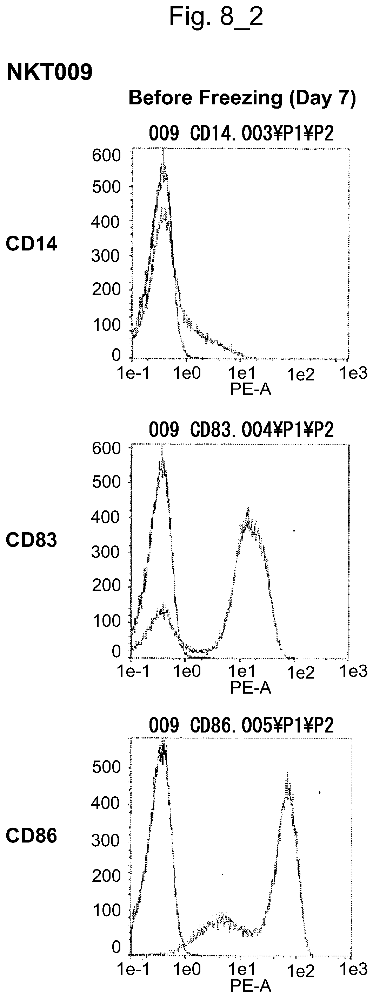

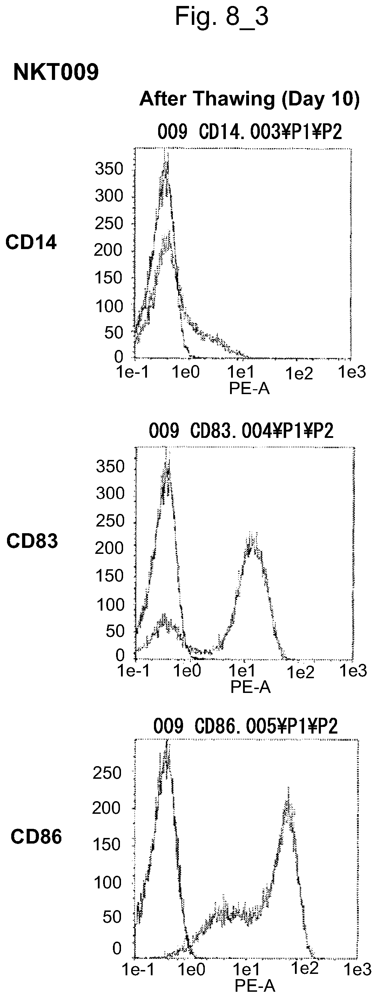

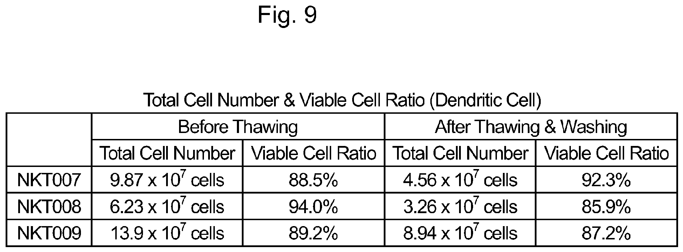

FIGS. 6 to 8 show the results upon analyzing the CD14, CD83, and CD86 on the cell surface with a flow cytometer on Day 0, before freezing (after induction of differentiation), and after freezing and thawing for the cells of three healthy individuals (ID: NKT007, NKT008, and NKT009). FIG. 9 shows the total number of cells and the viable cell ratio before freezing and after thawing/washing for the group of experiments corresponding to FIGS. 6 to 8. In each sample, there was no difference in the surface markers before and after freezing for the differentiation-induced cells, and the disappearance of CD14 and the strengthening of CD83 and CD86 were confirmed (FIGS. 6 to 8). Further, in each sample, there was no change in the viable cell ratio before and after freezing (FIG. 9).

### (Comparative Example)

Referring to the method disclosed in Non-Patent Literature 1, the following experiment was conducted using mononuclear cells obtained from three healthy individuals (ID: NKT001, NKT002, NKT003). On the first day (Day 0), the cell components were first isolated by apheresis. The cell components were layered in layers of 30 mL each in a LeucoSep, and centrifugation (3000 rpm, 20 min, r/t, Accel: 1, Decel: 1) was carried out. Thereafter, the mononuclear cell layer was collected in a 50 mL centrifuge tube, an equivalent amount of physiological salt solution was added thereto to create a suspension, and then centrifugation (1500 rpm, 5 min, r/t, Accel: 3, Decel: 2) was carried out. After removal of the supernatant, 30 mL of AlyS505N-0 (containing 100 U/mL IL-2) was then added to create a suspension, and the cell aggregate was removed using a cell strainer and the number of cells was measured. 2 × 10⁷ mononuclear cells were sampled (for Day 0) for use in flow cytometer measurement. The mononuclear cells were seeded into four 225 cm² suspension culture flasks in an amount of 6 × 10⁷ cells/60 mL, and a reagent(s) was added as shown below to the four flasks.
Flask 1: none
Flask 2: 800 U/mL GM-CSF
Flask 3: 100 ng/mL α-GalCer
Flask 4: 800 U/mL GM-CSF + 100 ng/mL α-GalCer

The flasks were kept still in an incubator (37°C, 5% CO₂), and the remaining cells were seeded into bags (with 2L of culture medium) obtained by connecting two 1L bags with ALyS505N-0 (containing 100 U/mL of IL-2, 800 U/mL of GM-CSF, and 100 ng/mL of α-GalCer) using a connecting tube. The seeded bags were kept still for 6 days in an incubator (37°C, 5% CO₂). The cell surface molecules of the above-mentioned cells for flow cytometer measurement were measured in the same manner as in Example 1.

On the second day (Day 2) after the start of culturing, 20 mL of culture liquid was collected from each flask, and the cell concentration and viable cell ratio were measured. Subsequently, the culture liquid was centrifuged (1500 rpm, 5 min, r/t, Accel: 3, Decel: 3), and then 1 mL thereof was further sampled and centrifuged (1500 rpm, 5 min, r/t, Accel: 3, Decel: 3). The cell surface molecules of the centrifuged cells were measured using a flow cytometer.

On the sixth day (Day 6) after the start of culturing, 20 mL was collected from each flask, and the cell concentration and viable cell ratio were measured. Subsequently, the culture liquid was centrifuged (1500 rpm, 5 min, r/t, Accel: 3, Decel: 3), and then 1 mL thereof was further sampled and centrifuged (1500 rpm, 5 min, r/t, Accel: 3, Decel: 3). The cell surface molecules of the centrifuged cells were measured using a flow cytometer.

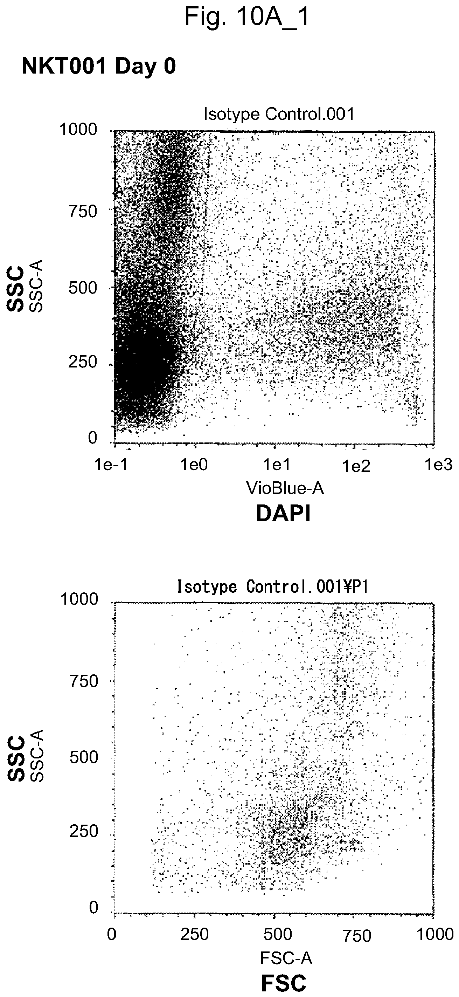

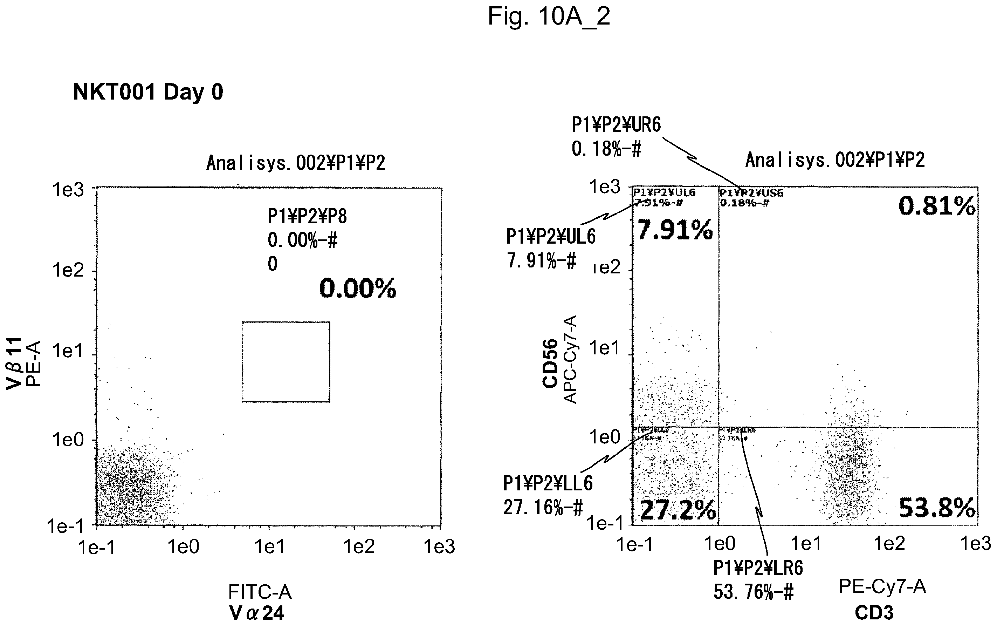

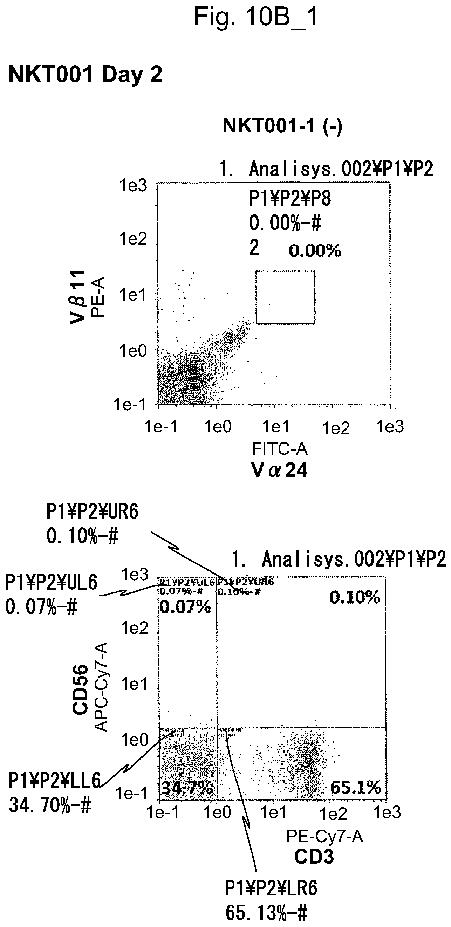

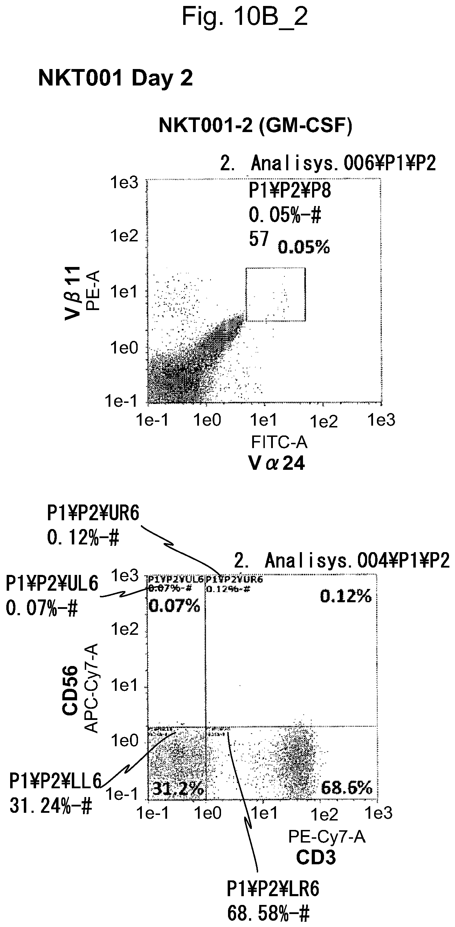

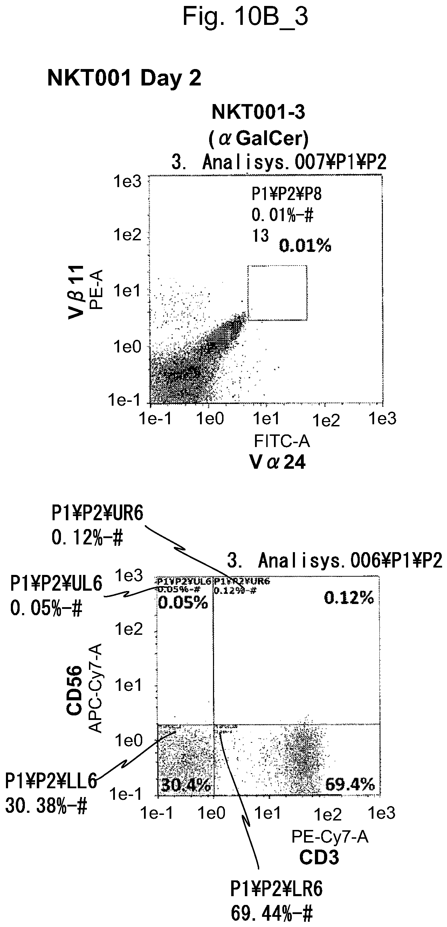

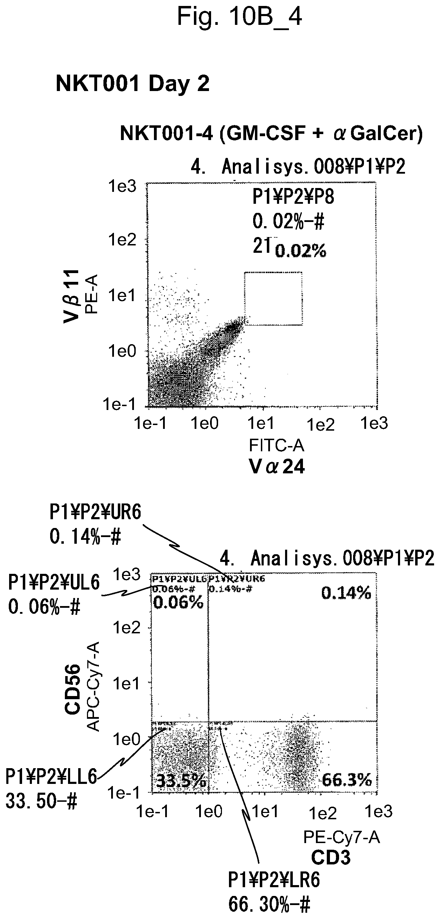

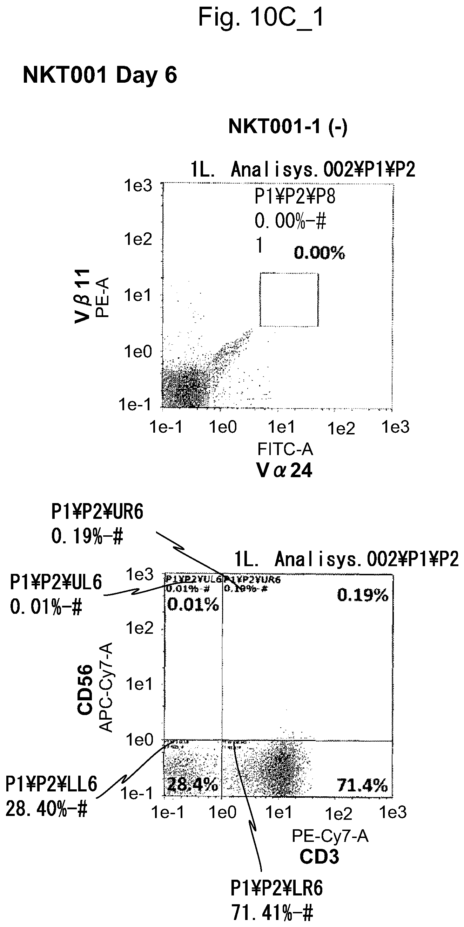

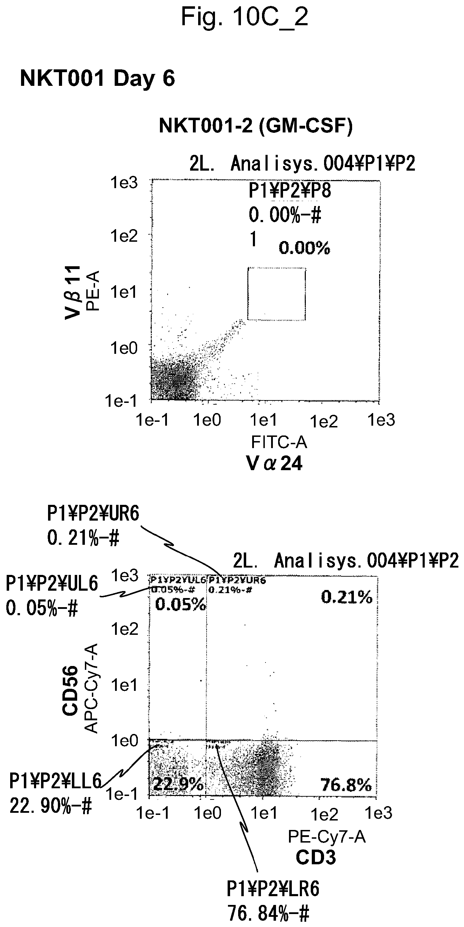

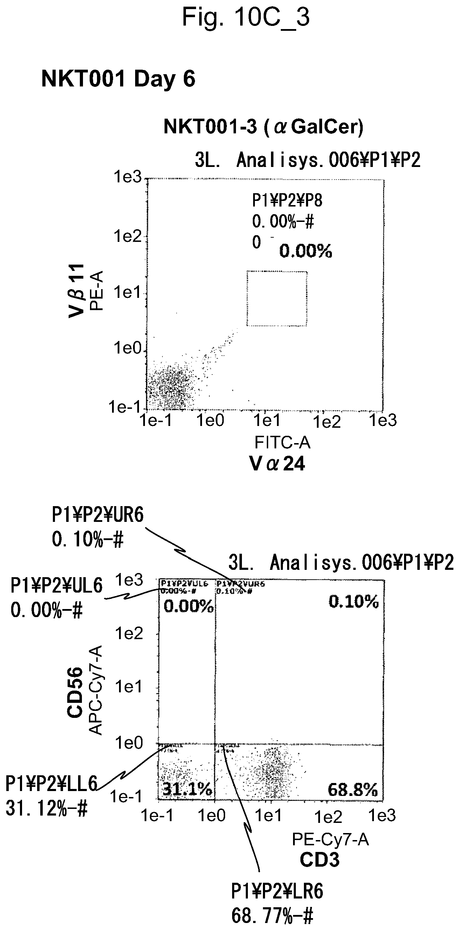

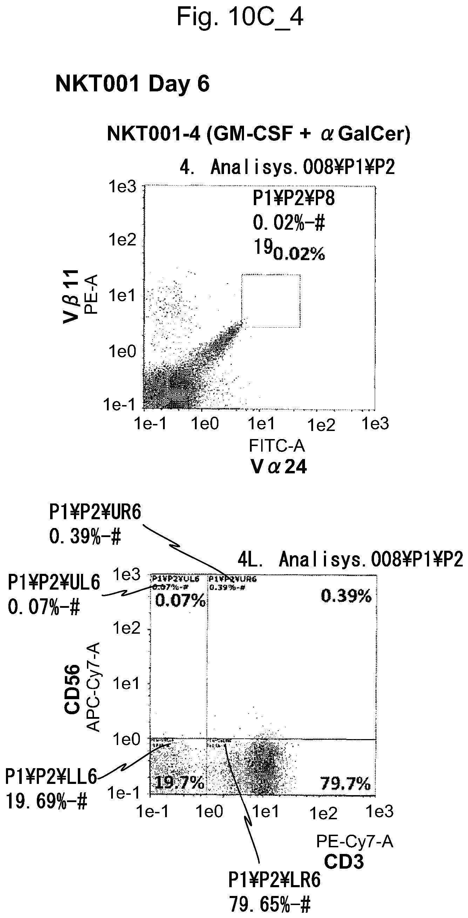

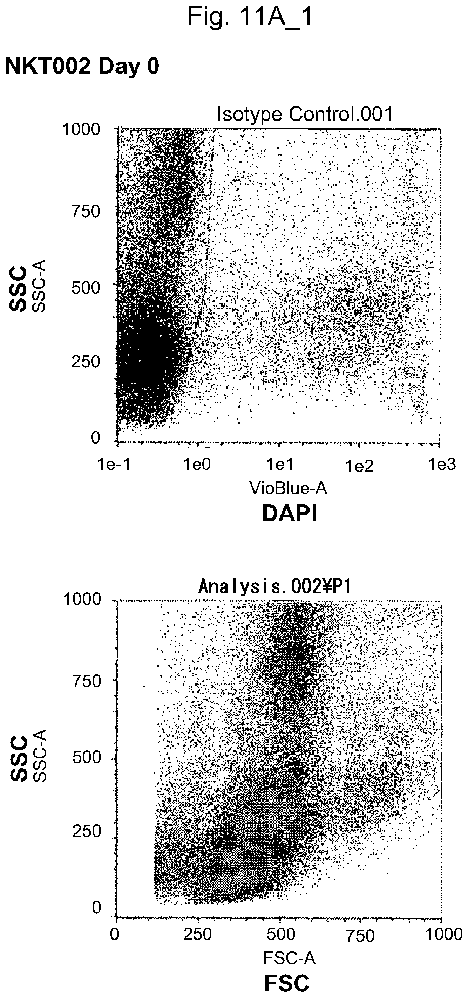

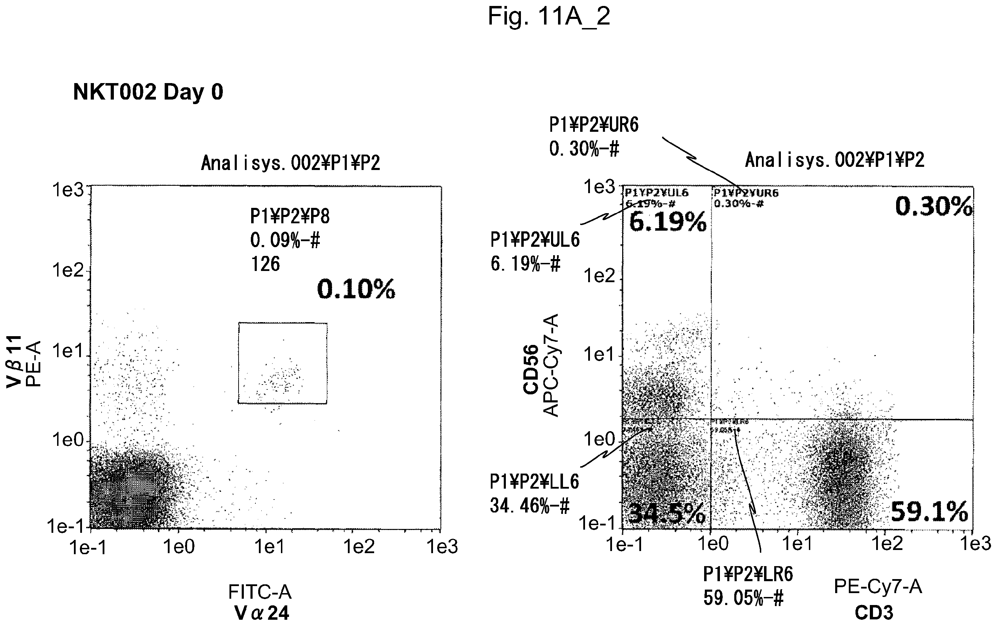

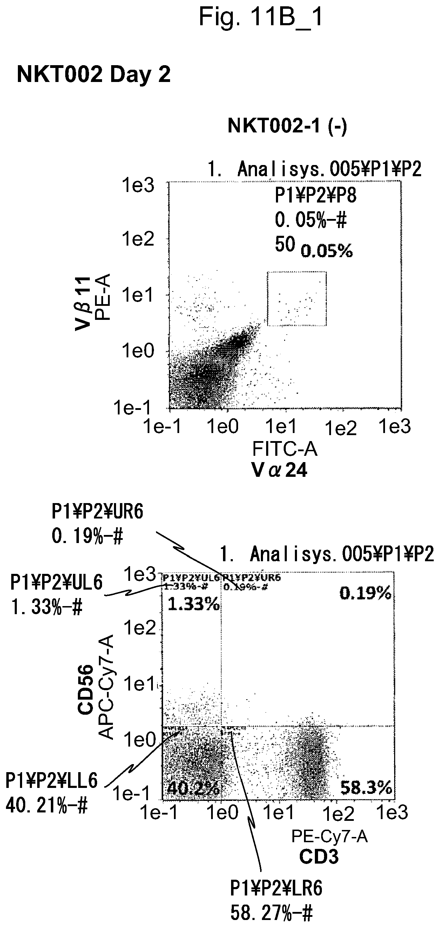

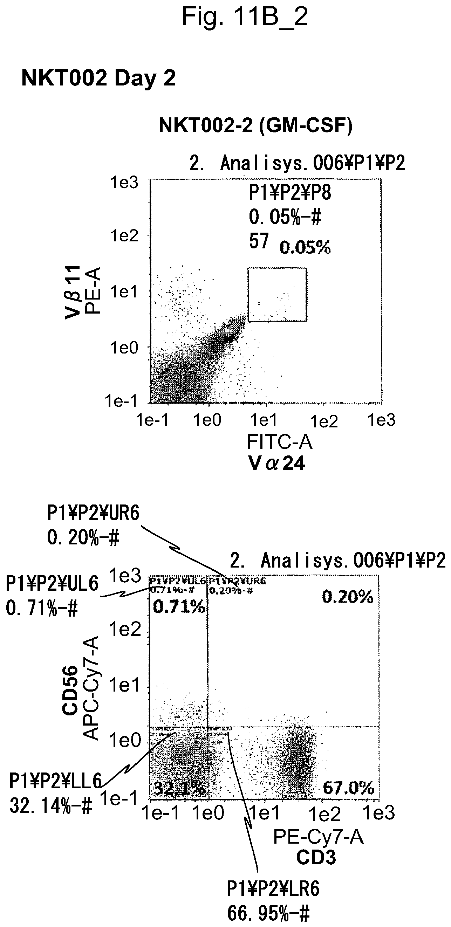

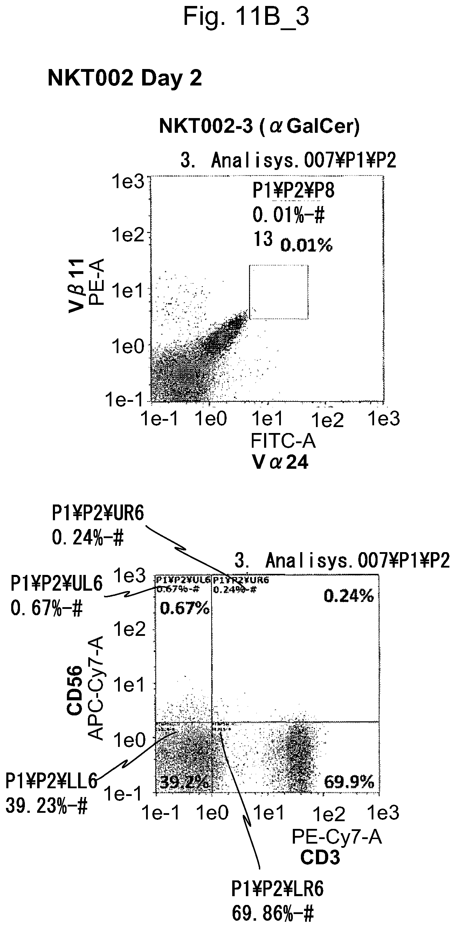

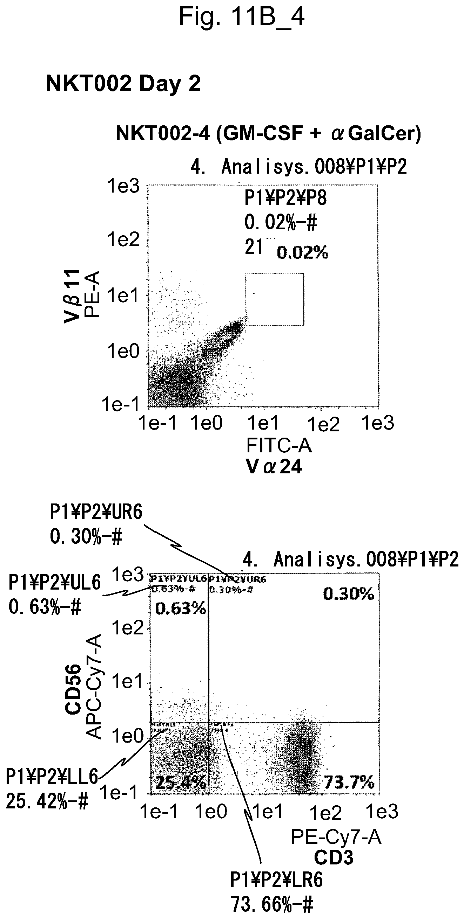

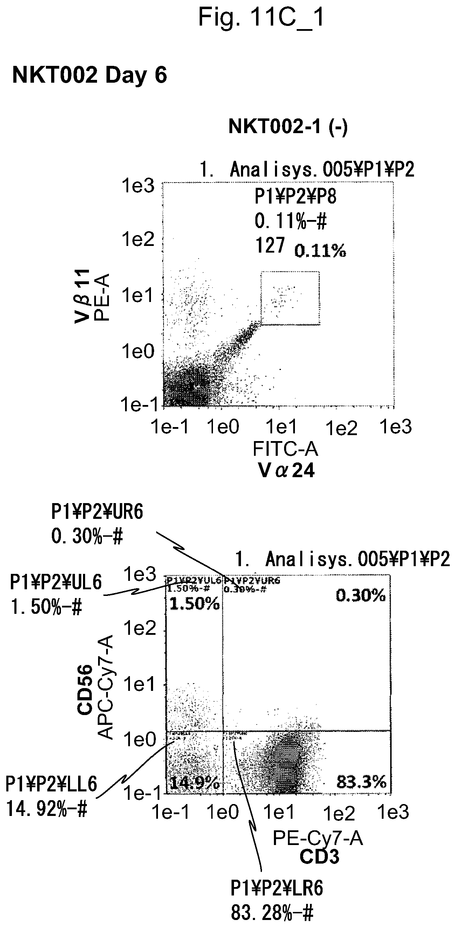

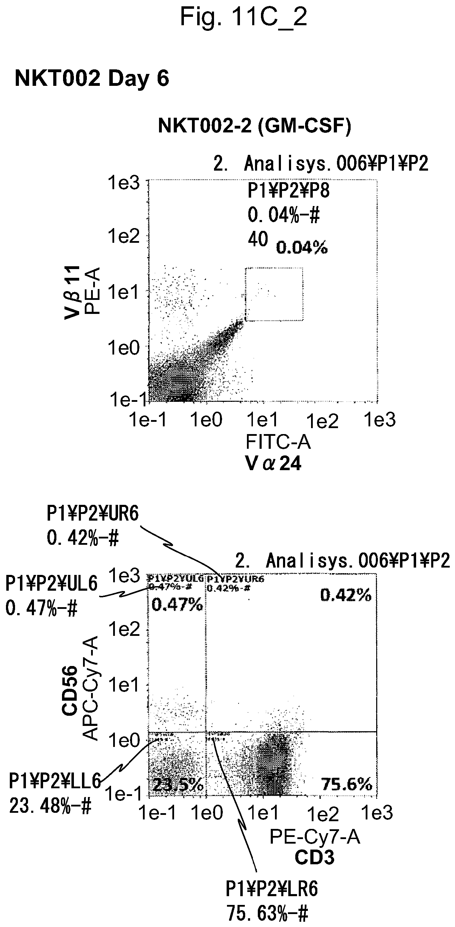

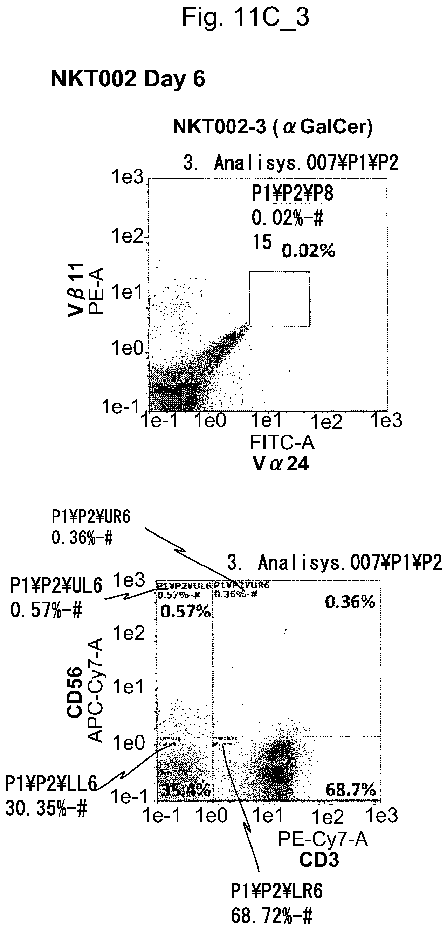

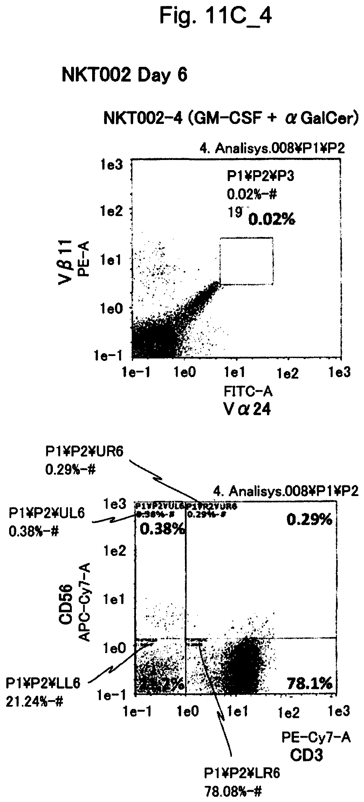

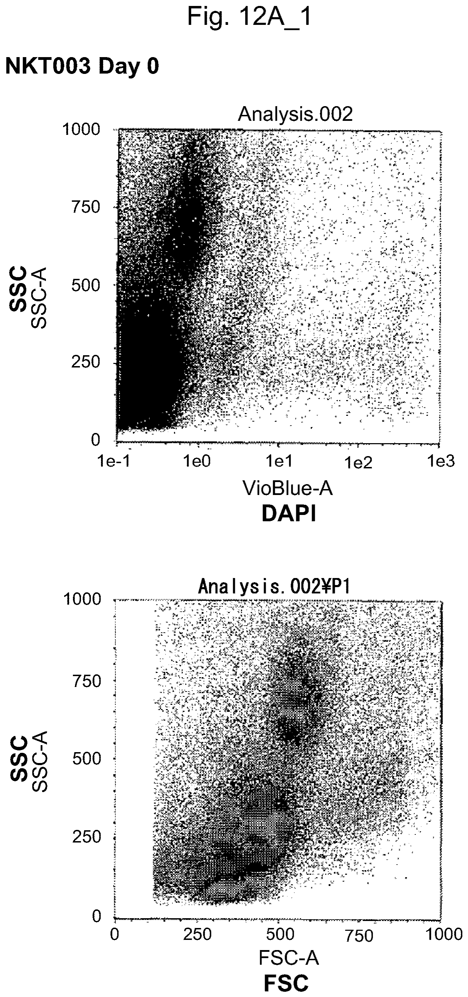

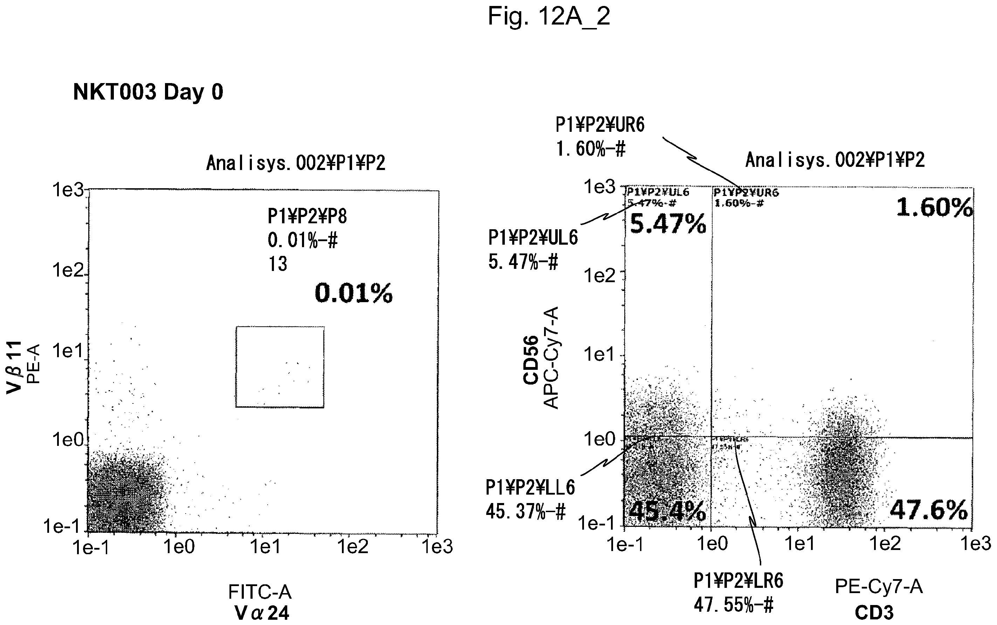

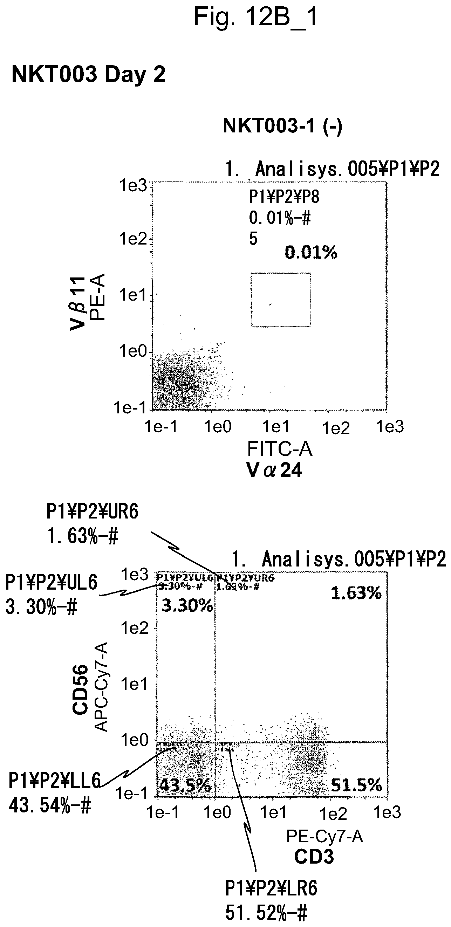

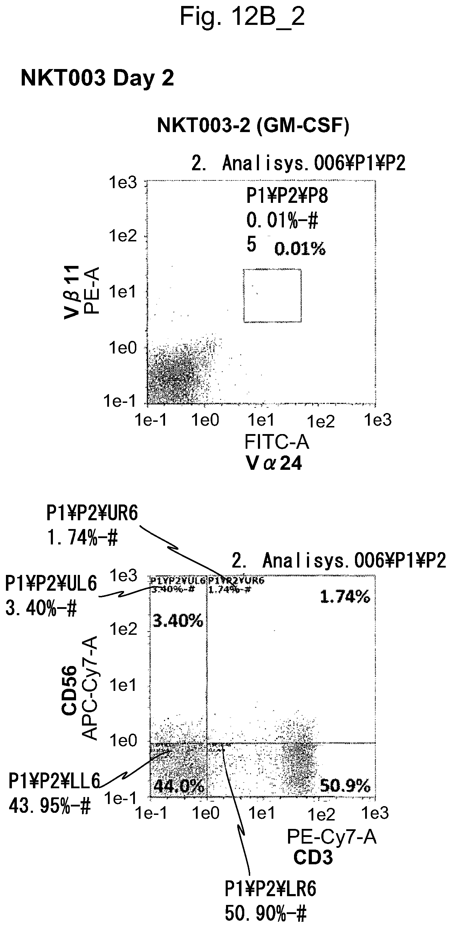

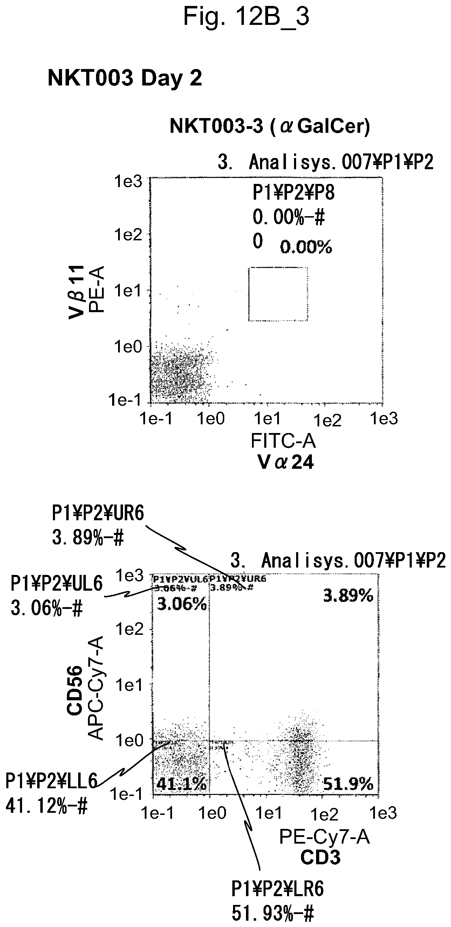

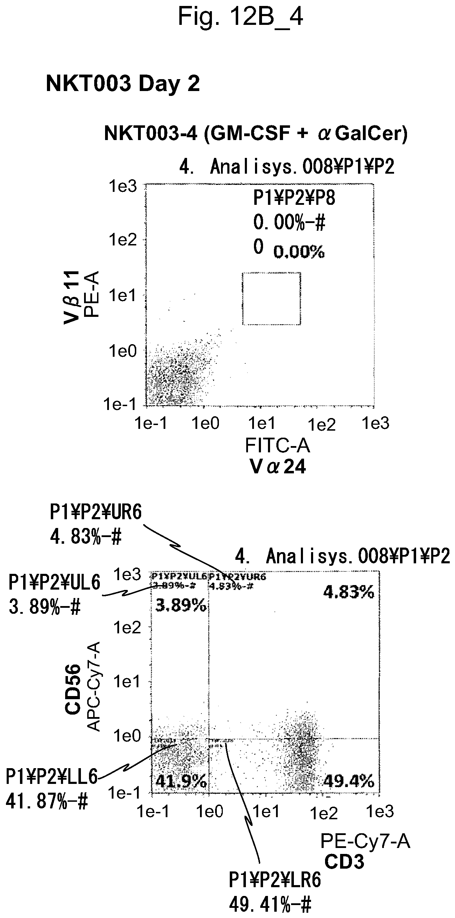

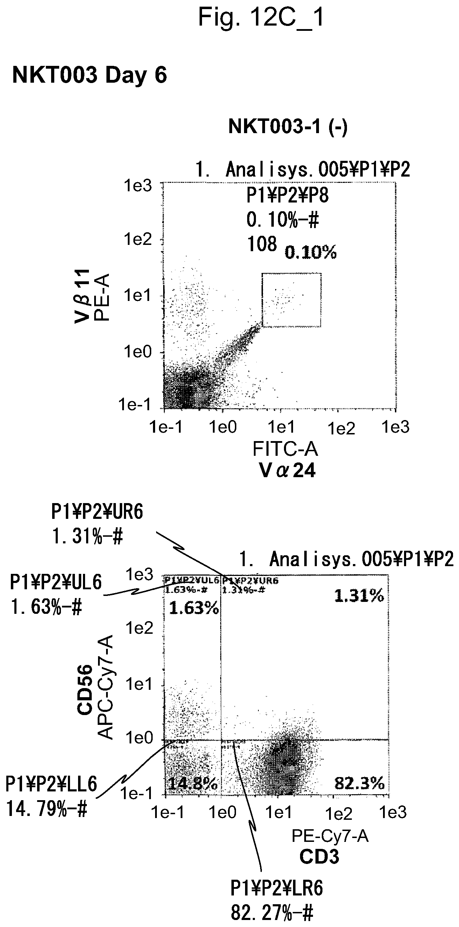

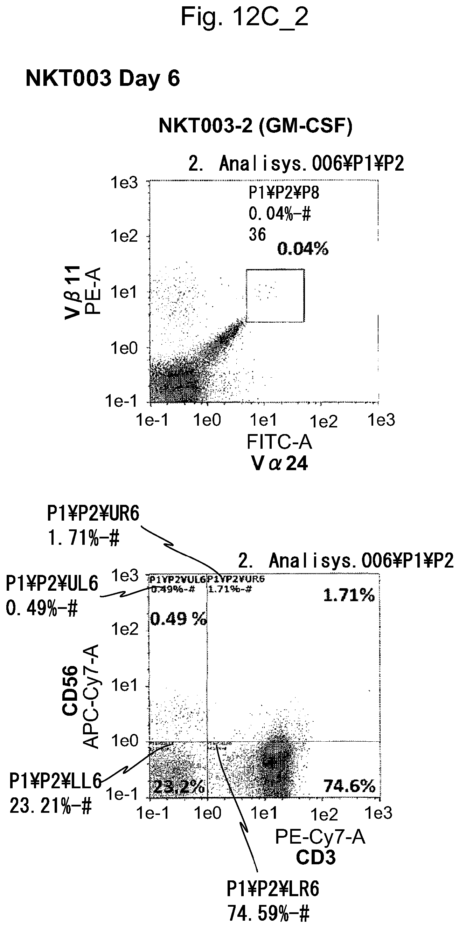

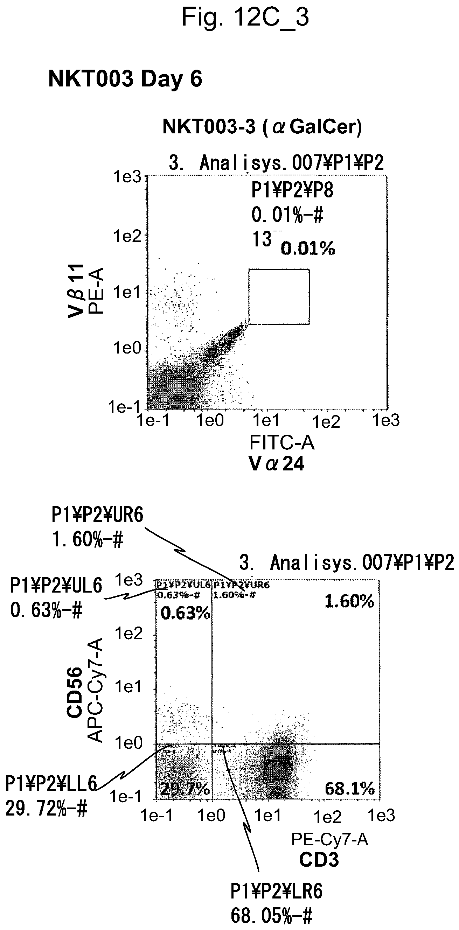

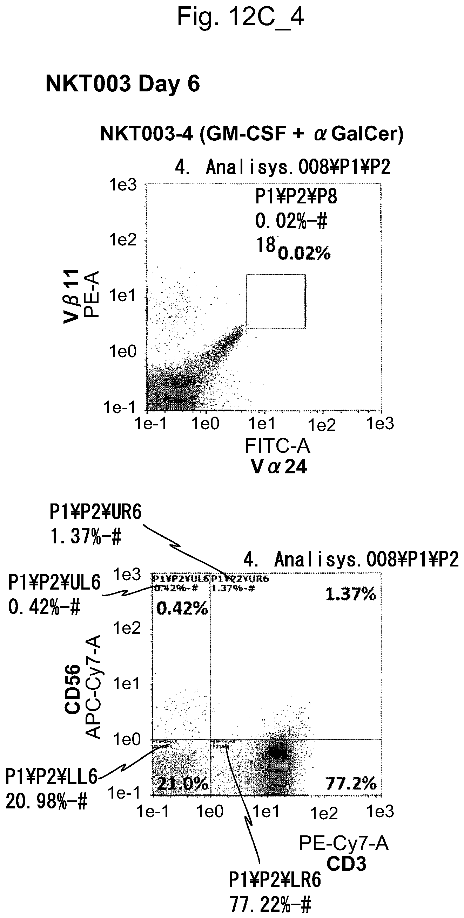

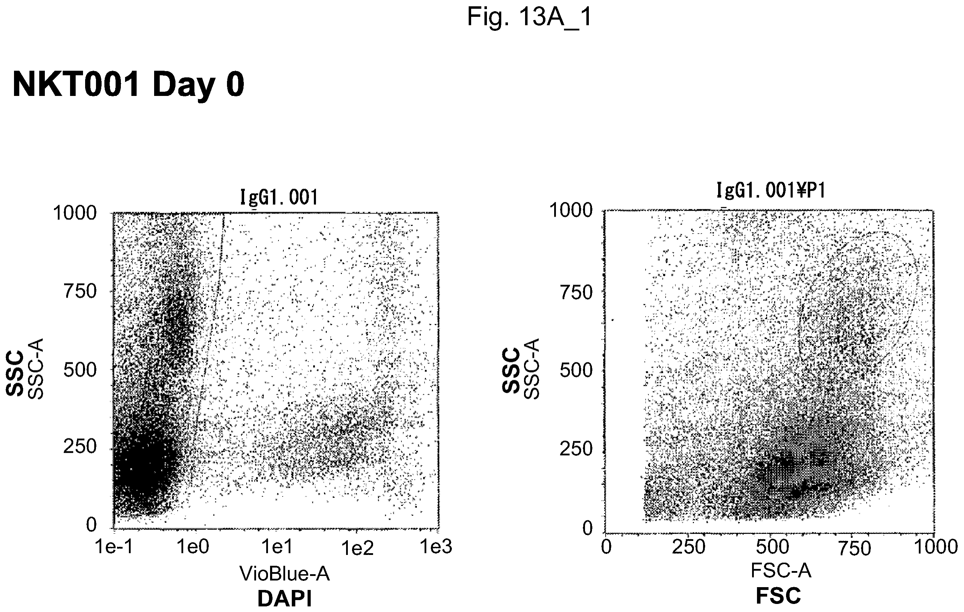

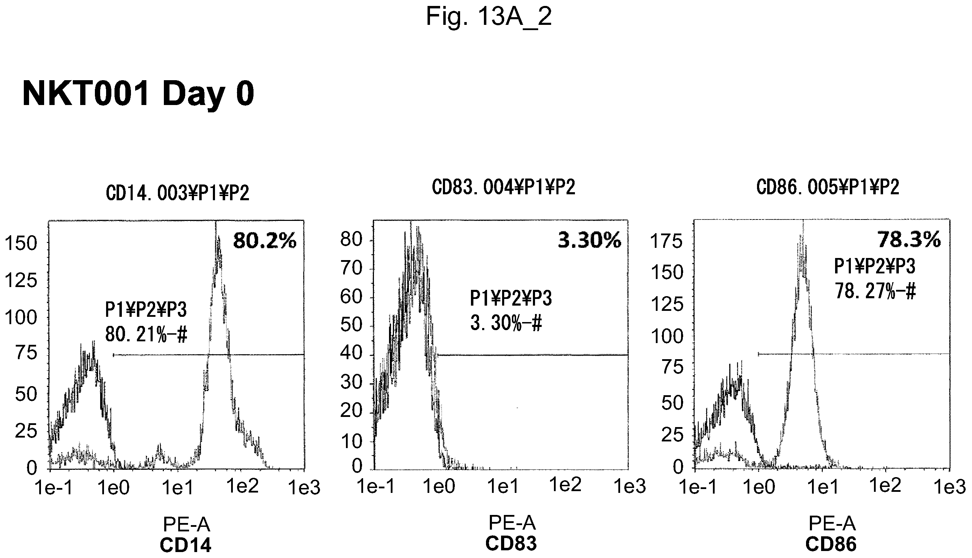

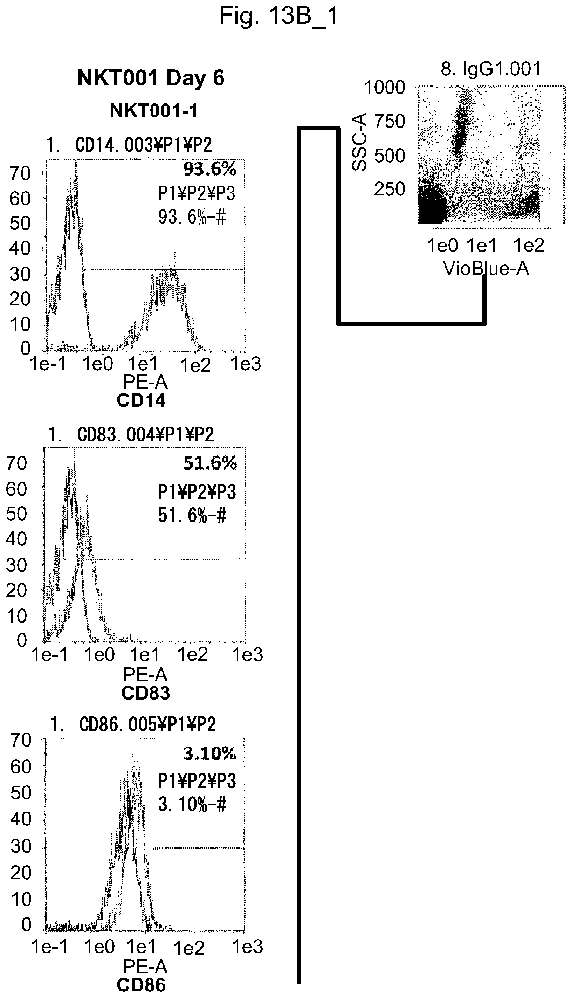

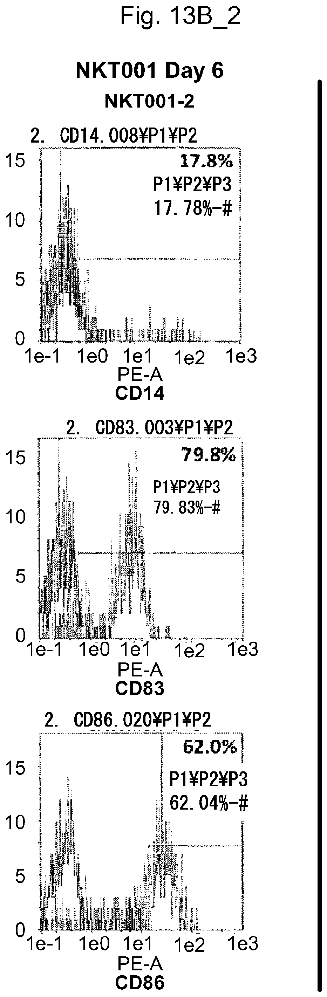

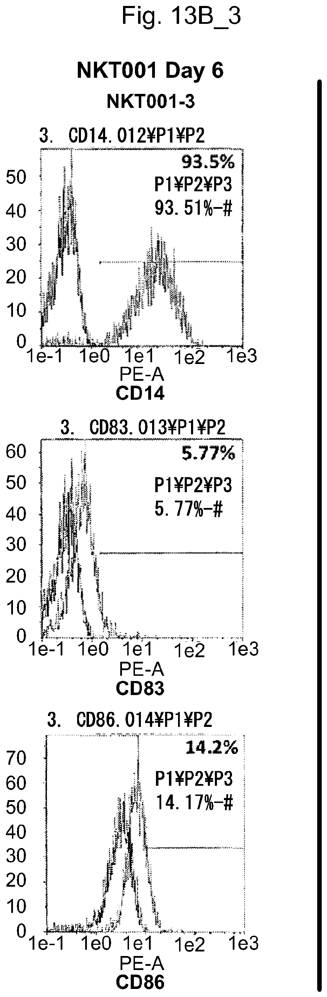

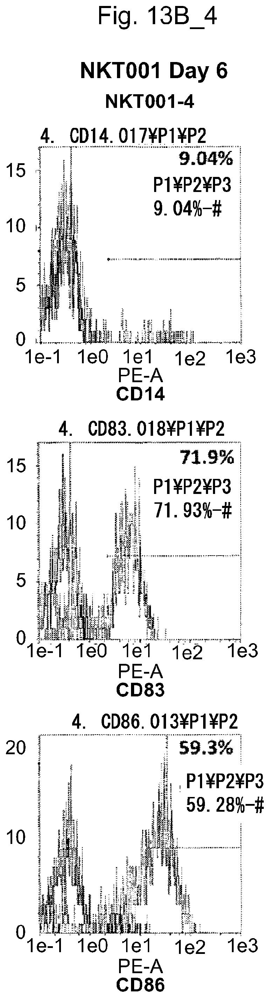

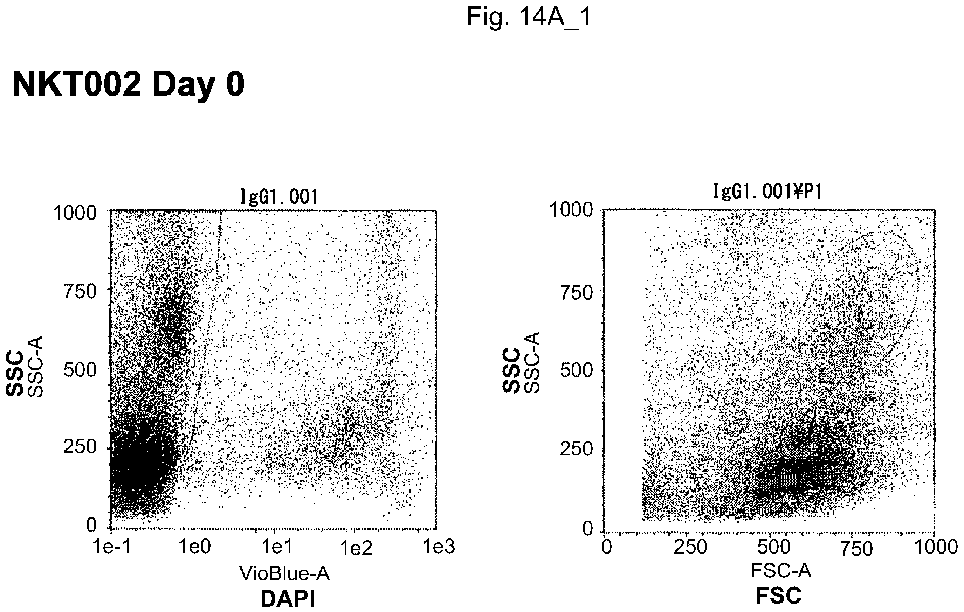

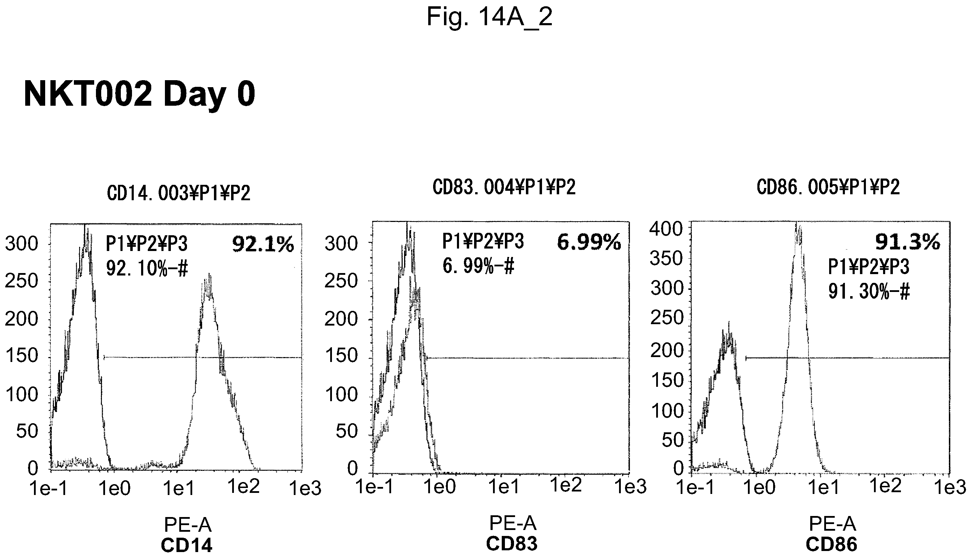

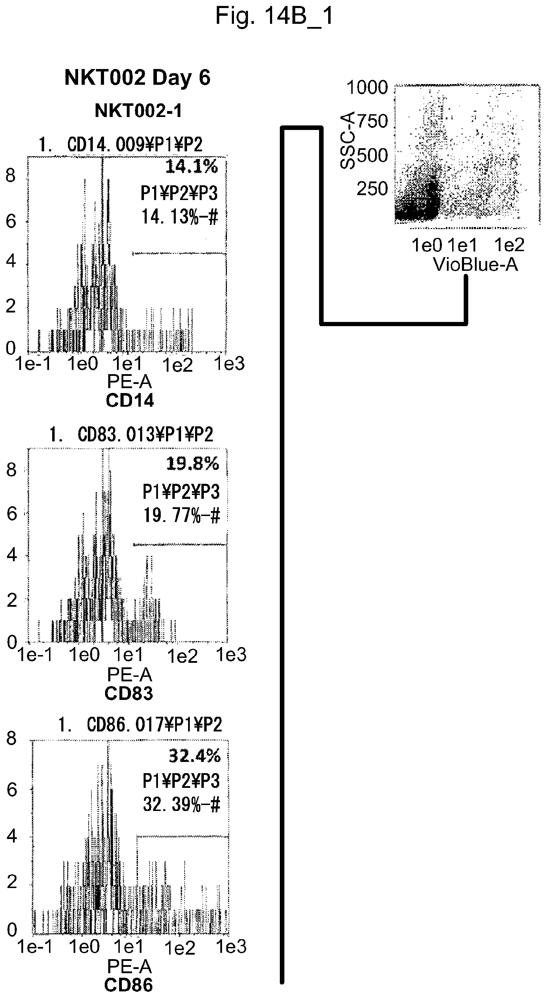

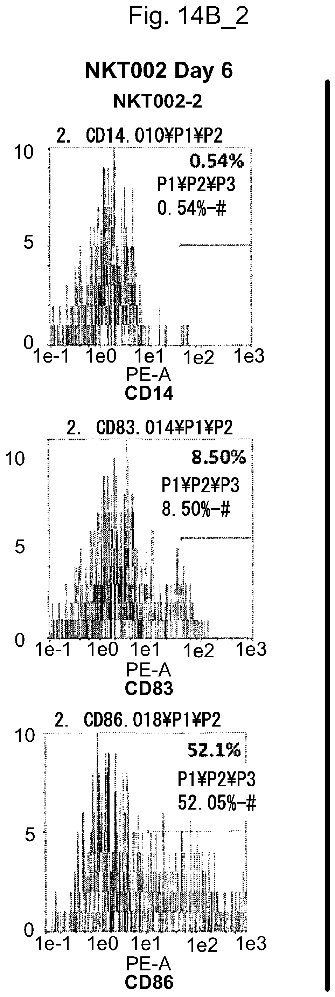

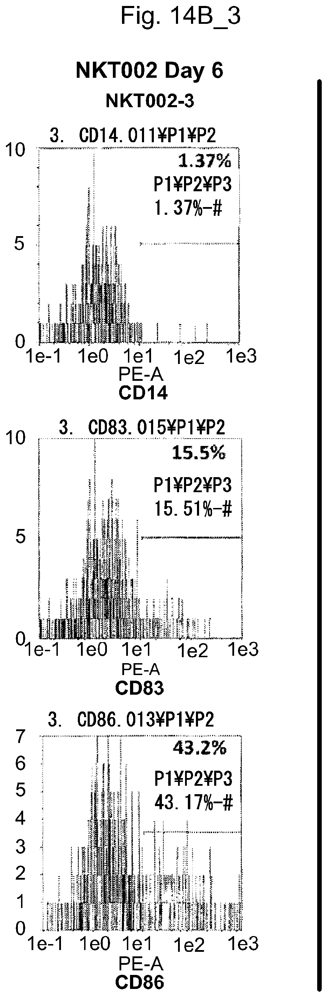

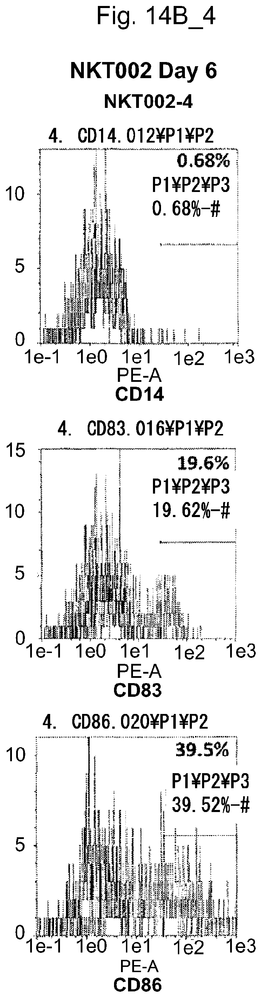

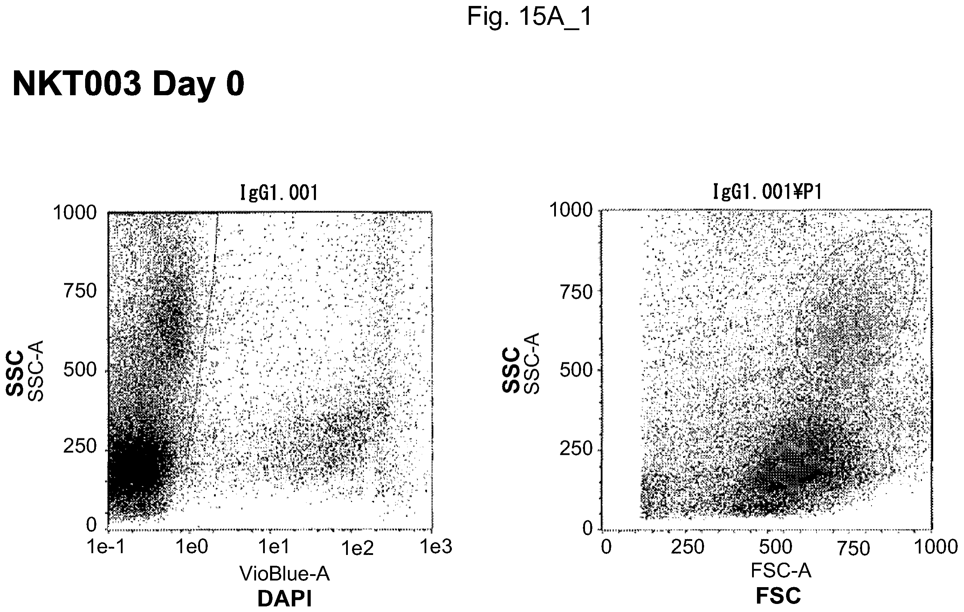

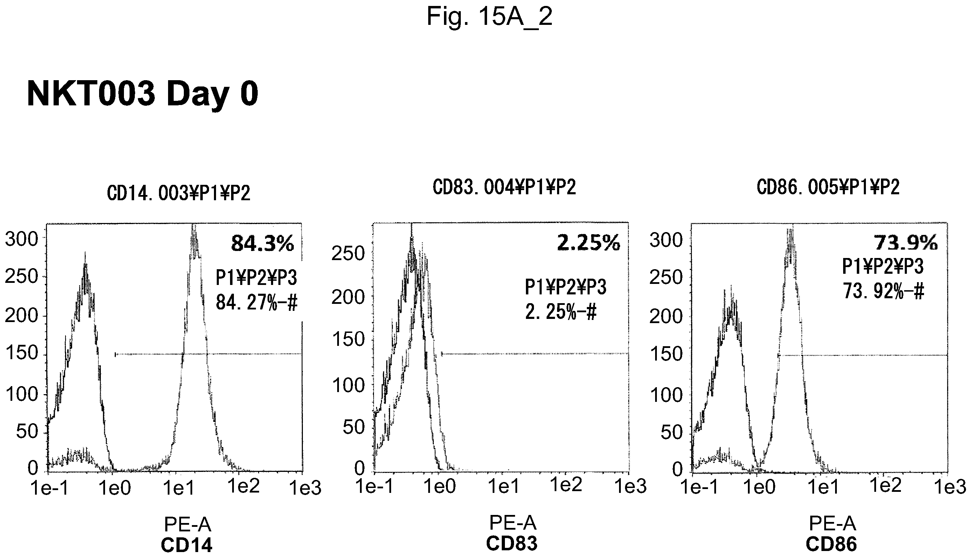

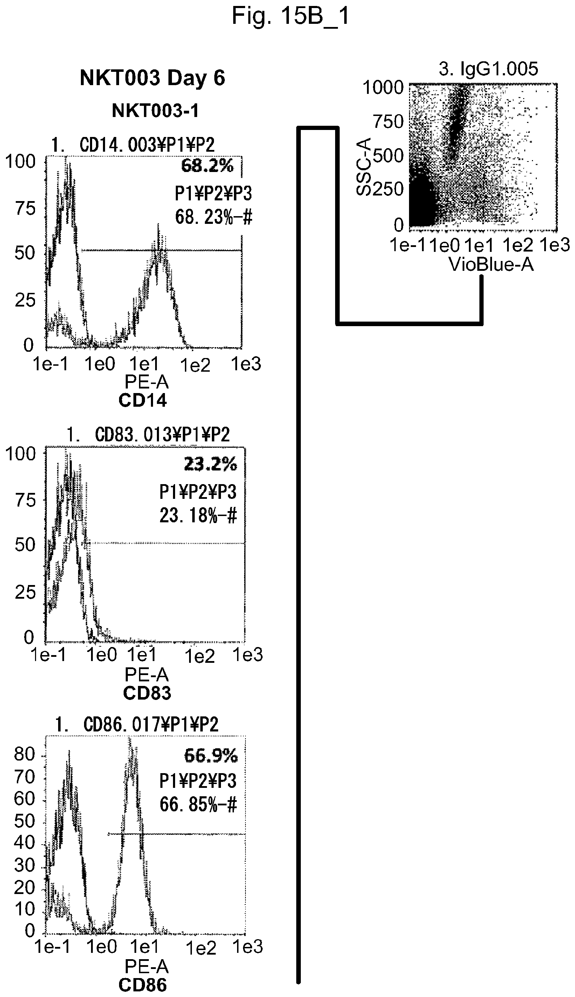

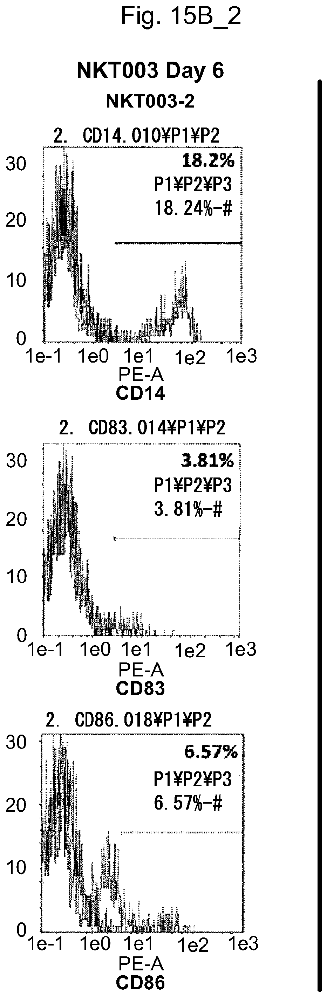

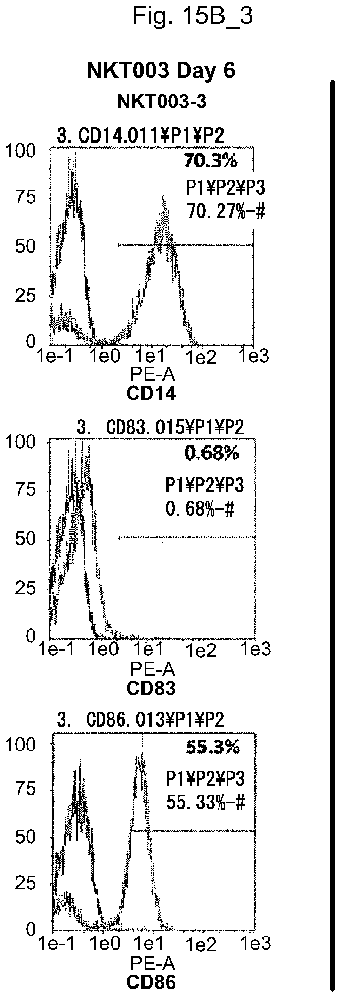

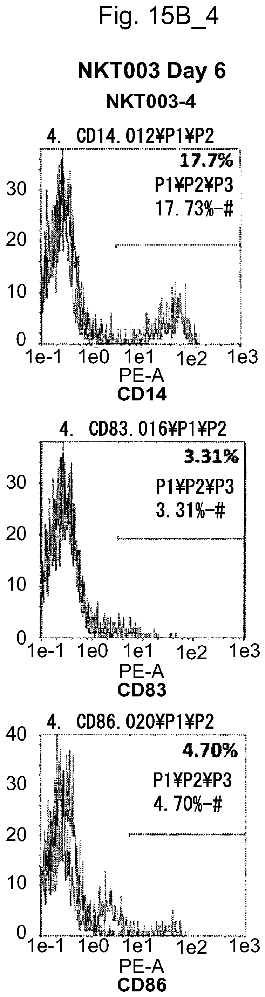

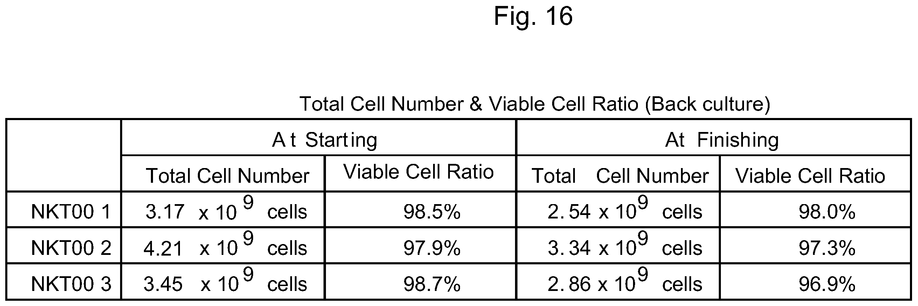

The NKT cells were analyzed with a flow cytometer on Day 0, Day 2, and Day 6, and the dendritic cells were analyzed with a flow cytometer on Day 0 and Day 6. The culture was carried out under conditions in which no cytokines were added, in which only GM-CSF was added, in which only α-GalCer was added, and in which GM-CSF and α-GalCer were both added. FIGS. 10(A) to 10(C), FIGS. 11(A) to 11(C), and FIGS. 12(A) to 12(C) show the results upon flow cytometer analysis of the NKT cells on Day 0, Day 2, and Day 6 for the three healthy individuals (ID: NKT001, NKT002, NKT003). FIGS. 13(A) and 13(B), FIGS. 14(A) and 14(B), and FIGS. 15(A) and 15(B) show the results upon flow cytometer analysis of the dendritic cells on Day 0 and Day 6 for the three healthy individuals (ID: NKT001, NKT002, NKT003). FIG. 16 shows the cell survival rate in this group of experiments. As shown in FIGS. 10(C), 11(C), and 12(C), no proliferation of NKT cells could be confirmed after 1 week of culture. There was no change in the total number of cells during this culture period, and there was no decrease in the viable cell ratio (FIG. 16). Further, as shown in FIGS. 13(B), 14(B), and 15(B), while a tendency for the monocytes to differentiate into dendritic cells was observed in each experiment group, clear results could not be obtained.

Given the above, it can be understood that in the methods of Examples 1 and 2, NKT cell-stimulating dendritic cells were obtained with good efficiency in the short period of about 1 week, and proliferation of NKT cells was achieved, as compared to the method of the Comparative Example. Further, in the method of the Comparative Example, liquid culture was used in the cell culture bags and the amount of culture medium that was used was greater than in the methods of Examples 1 and 2. In other words, the methods of Examples 1 and 2 used less culture medium (about one tenth) compared to the method of the Comparative Example, and thus are economical.

### INDUSTRIAL APPLICABILITY

The present invention is useful in the manufacture of NKT cell-stimulating dendritic cells and the manufacture of a cell composition containing NKT cell-stimulating dendritic cells and NKT cells, which are used in, for example, immunotherapy. According to these manufacturing methods, highly efficient, stable, and economical methods for manufacturing the dendritic cells and the cell composition can be provided, and thus the present invention is industrially useful.

## Claims

1. A method for manufacturing natural killer T (NKT) cell-stimulating dendritic cells, the method comprising the steps of:
(a1) an adhering step in which mononuclear cells are placed into a culture vessel and the culture vessel is kept still so as to allow some of the mononuclear cells to adhere to a bottom surface of the vessel;
(b1) a removal step in which suspended cells other than the cells which have adhered to the bottom surface of the culture vessel are removed;
(c1) a differentiation step in which both of IL-4 and granulocyte macrophage colony-stimulating factor are added into the culture vessel so as to cause monocytes among the cells which have adhered to the bottom surface to differentiate into immature dendritic cells;
(d1) a maturation step in which IL-4, granulocyte macrophage colony-stimulating factor, tumor necrosis factor α, and prostaglandin E2 are added into the culture vessel so as to mature the immature dendritic cells; and
(e1) an induction step in which α-galactosylceramide is added into the culture vessel so as to induce NKT cell-stimulating dendritic cells from the mature dendritic cells.

2. The method for manufacturing NKT cell-stimulating dendritic cells according to claim 1, wherein the concentration of the α-galactosylceramide in the induction step is 10 to 1,000 ng/mL.

3. The method for manufacturing NKT cell-stimulating dendritic cells according to claim 1 or 2, wherein the method further comprises a step for cryopreserving the obtained NKT cell-stimulating dendritic cells.

4. A method for manufacturing a cell composition containing NKT cell-stimulating dendritic cells and NKT cells, the method comprising:
(a2) an adhering step in which mononuclear cells are placed into a first culture vessel and the first culture vessel is kept still so as to allow some of the mononuclear cells to adhere to a bottom surface of the vessel;
(b2) a collection step in which suspended cells other than the cells which have adhered to the bottom surface of the vessel are collected and preserved;
(c2) a differentiation step in which both of IL-4 and granulocyte macrophage colony-stimulating factor are added into the first culture vessel so as to cause monocytes among the cells which have adhered to the bottom surface to differentiate into immature dendritic cells;
(d2) a maturation step in which IL-4, granulocyte macrophage colony-stimulating factor, tumor necrosis factor α, and prostaglandin E2 are added into the first culture vessel so as to mature the immature dendritic cells;
(e2) a dendritic cell induction step in which α-galactosylceramide is added into the first culture vessel so as to induce NKT cell-stimulating dendritic cells from the mature dendritic cells; and
(f2) an NKT cell induction step in which the NKT cell-stimulating dendritic cells and the preserved suspended cells are placed in a second culture vessel and the cells are cultured so as to induce NKT cells from some of the suspended cells.

5. The method for manufacturing a cell composition according to claim 4, wherein the concentration of the α-galactosylceramide in the induction step is 10 to 1,000 ng/mL.

6. The method for manufacturing a cell composition according to claim 4 or 5, wherein the method further comprises cryopreserving the cell composition containing the obtained NKT-stimulating cells and the NKT cells.

## Patentansprüche

1. Verfahren zur Herstellung von natürlichen Killer-T-Zellen (NKT) stimulierenden dendritischen Zellen, wobei das Verfahren die folgenden Schritte umfasst:
(a1) einen Adhäsionsschritt, bei dem mononukleare Zellen in ein Kulturgefäß gegeben werden und das Kulturgefäß stillgehalten wird, damit einige der mononuklearen Zellen an der Bodenfläche des Gefäßes haften bleiben;
(b1) einen Entfernungsschritt, bei dem suspendierte Zellen, die nicht an der Bodenfläche des Kulturgefäßes anhaften, entfernt werden;
(c1) einen Differenzierungsschritt, bei dem sowohl IL-4 als auch Granulozyten-Makrophagen-Kolonie-stimulierender Faktor in das Kulturgefäß gegeben werden, um die Monozyten unter den an der Bodenfläche anhaftenden Zellen zu unreifen dendritischen Zellen differenzieren zu lassen;
(d1) einen Reifungsschritt, in dem IL-4, Granulozyten-Makrophagen-Kolonie-stimulierender Faktor, Tumornekrosefaktor a und Prostaglandin E2 in das Kulturgefäß gegeben werden, um die unreifen dendritischen Zellen zu reifen; und
(e1) einen Induktionsschritt, bei dem α-Galactosylceramid in das Kulturgefäß gegeben wird, um aus den reifen dendritischen Zellen NKT-Zell-stimulierende dendritische Zellen zu induzieren.

2. Verfahren zur Herstellung von NKT-Zell-stimulierenden dendritischen Zellen nach Anspruch 1, wobei die Konzentration des α-Galactosylceramids im Induktionsschritt 10 bis 1.000 ng/ml beträgt.

3. Verfahren zur Herstellung von NKT-Zell-stimulierenden dendritischen Zellen nach Anspruch 1 oder 2, wobei das Verfahren ferner einen Schritt zum Kryokonservieren der erhaltenen NKT-Zell-stimulierenden dendritischen Zellen umfasst.

4. Verfahren zur Herstellung einer Zellzusammensetzung, die NKT-Zell-stimulierende dendritische Zellen und NKT-Zellen enthält, wobei das Verfahren umfasst:
(a2) einen Adhäsionsschritt, in dem mononukleare Zellen in ein erstes Kulturgefäß gegeben werden und das erste Kulturgefäß stillgehalten wird, damit einige der mononuklearen Zellen an der Bodenfläche des Gefäßes anhaften können;
(b2) einen Sammelschritt, bei dem suspendierte Zellen, die nicht an der Bodenfläche des Gefäßes anhaften, gesammelt und konserviert werden;
(c2) einen Differenzierungsschritt, bei dem sowohl IL-4 als auch Granulozyten-Makrophagen-Kolonie-stimulierender Faktor in das erste Kulturgefäß gegeben werden, um die Monozyten unter den an der Bodenfläche anhaftenden Zellen zu unreifen dendritischen Zellen differenzieren zu lassen;
(d2) einen Reifungsschritt, in dem IL-4, Granulozyten-Makrophagen-Kolonie-stimulierender Faktor, Tumornekrosefaktor a und Prostaglandin E2 in das erste Kulturgefäß gegeben werden, um die unreifen dendritischen Zellen zu reifen;
(e2) einen Schritt zur Induktion dendritischer Zellen, bei dem α-Galactosylceramid in das erste Kulturgefäß gegeben wird, um aus den reifen dendritischen Zellen NKT-Zellen-stimulierende dendritische Zellen zu induzieren; und
(f2) einen NKT-Zell-Induktionsschritt, bei dem die NKT-Zell-stimulierenden dendritischen Zellen und die konservierten suspendierten Zellen in ein zweites Kulturgefäß gegeben werden und die Zellen kultiviert werden, um aus einigen der suspendierten Zellen NKT-Zellen zu induzieren.

5. Verfahren zur Herstellung einer Zellzusammensetzung nach Anspruch 4, wobei die Konzentration des α-Galactosylceramids im Induktionsschritt 10 bis 1.000 ng/ml beträgt.

6. Verfahren zur Herstellung einer Zellzusammensetzung nach Anspruch 4 oder 5, wobei das Verfahren ferner das Kryokonservieren der Zellzusammensetzung umfasst, die die erhaltenen NKT-stimulierenden Zellen und die NKT-Zellen enthält.

## Revendications

1. Un procédé de fabrication de cellules dendritiques stimulant les cellules T tueuses naturelles (NKT), le procédé comprenant les étapes suivantes :
(a1) une étape d'adhérence dans laquelle des cellules mononucléaires sont placées dans un récipient de culture et le récipient de culture est maintenu immobile afin de permettre à certaines des cellules mononucléaires d'adhérer à la surface inférieure du récipient ;
(b1) une étape d'élimination dans laquelle les cellules en suspension autres que les cellules qui ont adhéré à la surface inférieure du récipient de culture sont éliminées ;
(c1) une étape de différenciation dans laquelle de l'IL-4 et du facteur de stimulation des colonies de granulocytes et de macrophages sont ajoutés dans le récipient de culture afin de provoquer la différenciation des monocytes parmi les cellules qui ont adhéré à la surface inférieure en cellules dendritiques immatures ;
(d1) une étape de maturation dans laquelle de l'IL-4, du facteur de stimulation des colonies de granulocytes et de macrophages, du facteur de nécrose tumorale α et de la prostaglandine E2 sont ajoutés dans le récipient de culture afin de faire mûrir les cellules dendritiques immatures ; et
(e1) une étape d'induction dans laquelle de l'α-galactosylcéramide est ajouté dans le récipient de culture afin d'induire des cellules dendritiques stimulant les cellules NKT à partir des cellules dendritiques matures.

2. Le procédé de fabrication de cellules dendritiques stimulant les cellules NKT selon la revendication 1, dans lequel la concentration en α-galactosylcéramide dans l'étape d'induction est de 10 à 1000 ng/mL.

3. Le procédé de fabrication de cellules dendritiques stimulant les cellules NKT selon la revendication 1 ou 2, dans lequel le procédé comprend en outre une étape de cryoconservation des cellules dendritiques stimulant les cellules NKT obtenues.

4. Un procédé de fabrication d'une composition cellulaire contenant des cellules dendritiques stimulant les cellules NKT et des cellules NKT, le procédé comprenant :
(a2) une étape d'adhérence dans laquelle les cellules mononucléaires sont placées dans un premier récipient de culture et le premier récipient de culture est maintenu immobile afin de permettre à certaines des cellules mononucléaires d'adhérer à la surface inférieure du récipient ;
(b2) une étape de collecte dans laquelle les cellules en suspension autres que les cellules qui ont adhéré à la surface inférieure du récipient sont collectées et conservées ;
(c2) une étape de différenciation dans laquelle de l'IL-4 et du facteur de stimulation des colonies de granulocytes et de macrophages sont ajoutés dans le premier récipient de culture afin de provoquer la différenciation des monocytes parmi les cellules qui ont adhéré à la surface inférieure en cellules dendritiques immatures ;
(d2) une étape de maturation dans laquelle de l'IL-4, du facteur de stimulation des colonies de granulocytes et de macrophages, du facteur de nécrose tumorale α et de la prostaglandine E2 sont ajoutés dans le premier récipient de culture afin de faire mûrir les cellules dendritiques immatures ;
(e2) une étape d'induction des cellules dendritiques dans laquelle de l'α-galactosylcéramide est ajouté dans le premier récipient de culture afin d'induire des cellules dendritiques stimulant les cellules NKT à partir des cellules dendritiques matures ; et
(f2) une étape d'induction des cellules NKT dans laquelle les cellules dendritiques stimulant les cellules NKT et les cellules en suspension conservées sont placées dans un deuxième récipient de culture et les cellules sont cultivées de manière à induire des cellules NKT à partir de certaines des cellules en suspension.

5. Le procédé de fabrication d'une composition cellulaire selon la revendication 4, dans lequel la concentration en α-galactosylcéramide dans l'étape d'induction est de 10 à 1000 ng/mL.

6. Le procédé de fabrication d'une composition cellulaire selon la revendication 4 ou 5, dans lequel le procédé comprend en outre la cryoconservation de la composition cellulaire contenant les cellules stimulant les NKT et les cellules NKT obtenues.
